# NEUE EUROPÄISCHE PATENTSCHRIFT

(11) **EP 1 858 480 B3**
(45) Veröffentlichungstag dieser Patentschrift: **06.05.2020**
(45) Hinweis auf die Patenterteilung: 29.05.2013
(21) Anmeldenummer: 06723302.3
(22) Anmeldetag: 09.03.2006
(51) Int. Cl.: A61K 8/37, A61Q 19/00, A61Q 5/00, C07C 69/24

(54) **KOSMETISCHE ZUSAMMENSETZUNGEN ENTHALTEND ESTER AUF BASIS VON 2- PROPYLHEPTANOL**
COSMETIC COMPOSITIONS COMPRISING ESTER BASED ON 2-PROPYLHEPTANOL
COMPOSITION COSMETIQUE COMPRENANT UN ESTER DE 2-PROPYLHEPTANOL

(30) Priorität: 17.03.2005 DE 102005012300; 10.06.2005 EP 05012510
(43) Veröffentlichungstag der Anmeldung: 28.11.2007
(73) Patentinhaber: Cognis IP Management GmbH, 40589 Düsseldorf (DE)
(72) Erfinder: DIERKER, Markus, 40597 Düsseldorf (DE); WEICHOLD, Catherine, 52062 Aachen (DE); ZANDER, Lars, 41569 Rommerskirchen (DE); ALTHAUS, Stefanie, 51061 Köln (DE); PRINZ, Daniela, 41542 Dormagen (DE)
(86) Internationale Anmeldenummer: PCT/EP2006/002148
(87) Internationale Veröffentlichungsnummer: WO 2006/097235

(56) Entgegenhaltungen:
- WO-A-01/14309
- DE-A1- 10 305 562
- DE-A1- 19 742 275
- DE-A1- 19 926 671
- DE-B- 1 138 756
- US-A1- 2004 014 865
- PATENT ABSTRACTS OF JAPAN Bd. 1995, Nr. 09, 31. Oktober 1995 (1995-10-31) & JP 07 157615 A (MITSUBISHI CHEM CORP), 20. Juni 1995 (1995-06-20)

## Beschreibung

### Gebiet der Erfindung

Die Erfindung betrifft die Verwendung von Estern des 2-Proplyheptanols in kosmetischen und/oder pharmazeutischen Zubereitungen sowie spezifische Ester und ein Verfahren zu deren Herstellung.

### Stand der Technik

Im Bereich kosmetischer Emulsionen für die Haut- und Haarpflege werden vom Verbraucher eine Vielzahl von Anforderungen gestellt: Abgesehen von den reinigenden und pflegenden Effekten, die den Anwendungszweck bestimmen, wird Wertauf so unterschiedliche Parameter wie höchstmögliche dermatologische Verträglichkeit, gute rückfettende Eigenschaften, elegantes Erscheinungsbild, optimaler sensorischer Eindruck und Lagerstabilität gelegt.

Zubereitungen, die zur Reinigung und Pflege der menschlichen Haut und der Haare eingesetzt werden, enthalten in der Regel neben einer Reihe von oberflächenaktiven Substanzen, vor allem Ölkörper und Wasser. Als Ölkörper/Emollients werden beispielsweise Kohlenwasserstoffe, Esteröle sowie pflanzliche und tierische Öle/Fette/Wachse eingesetzt. Um die hohen Anforderungen des Marktes bezüglich sensorischer Eigenschaften und optimaler dermatologischerVerträglichkeit zu erfüllen, werden kontinuierlich neue Ölkörper und Emulgator-Gemische entwickelt und getestet. Die Verwendung von Esterölen in der Kosmetik ist seit langem bekannt. Wegen ihrer Bedeutung werden auch kontinuierlich neue Verfahren zu deren Herstellung entwickelt. Insbesondere verzweigte Esteröle vermitteln ein "leichteres" Hautgefühlt und werden daher intensiv untersucht. Der Einsatz von 2-Methyl-1,3-propanediolmonoestern ist beispielsweise Gegenstand der DE 101 60 681, der Einsatz von 2-Methyl-1,3-propanedioldiestern wird in der DE 10160 682 beschrieben.

Aufgabe der vorliegenden Erfindung war es, neue, vorzugsweise bei 20 °C flüssige Esteröle für kosmetische Applikationen zur Verfügung zu stellen, die bezüglich der sensorischen Eigenschaften (Leichtigkeit, "nicht-fettendes Hautgefühl", Weichheit, Spreitvermögen, Absorption, Verteilbarkeit, Öligkeit) über ein verbessertes Profil verfügen und in eine Vielzahl kosmetischer Formulierungen einarbeitbar sind. Hierbei waren auch die Hydrolysestabilität der Ester sowie die Formulierbarkeit der Ester bei niedrigem pH-Wert von Interesse. Weiterhin sollten die Ester sowohl in W/O als auch in O/W Formulierungen einarbeitbar sein. Weiterhin sollten die Ester insbesondere mit kristallinen UV-Filtem, Pigmenten, Antiperspirantien Salzen sowie Silikonen kompatibel sein. Überraschenderweise wurde gefunden, dass Ester des 2-Propylheptanols zu sensorisch leichten Produkten führen. Einige dieser Ester und deren Herstellung sind aus der DE 103 05 562 bekannt, allerdings für einen völlig anderen Applikationszweck, als Polymeradditive.

### Beschreibung der Erfindung

Gegenstand der Erfindung ist die Verwendung von Estern des 2-Propylheptanols mit linearen oder verzweigten, gesättigten oder ungesättigten C4-C36-Carbonsäuren - oder C4-C36-Dicarbonsäuren in kosmetischen und/oder pharmazeutischen Zubereitungen.

Überaschenderweise sind Ester mit Propylverzweigung in der Alkylkette besonders gut für kosmetische Formulierungen geeignet, insbesondere für Formulierungen, bei denen es auf ein "leichtes" Hautgefühl ankommt. Die Ester lassen sich sehr gut in verschiedene Formulierungen einarbeiten. Je nach Kettenlänge, Verzweigung und Anzahl der Doppelbindungen erhält man flüssige Substanzgemische, die sich entsprechend als Ölkörper oder Konsistenzgeber eignen. Erfindungsgemäß kann ein einziger 2-Propylheptyl-C4-C36-Carbonsäureester oder 2-Propylheptyl-C4-C36-Dicarbonsäureester eingesetzt werden oder ein beliebiges Gemisch.

Gegenstand der Erfindung ist insbesondere die Verwendung von Estern des 2-Propylheptanols mit linearen oder verzweigten, gesättigten oder ungesättigten C4-C36-Carbonsäuren - oder C4-C36-Dicarbonsäuren in kosmetischen und/oder pharmazeutischen Zubereitungen zur Benetzung oder Imprägnierung oder Beschichtung von Gebrauchs- und/oder Hygienetüchem, die zur Körperreinigung und/oder zur Körperpflege eingesetzt werden.

In einer bevorzugten Ausführungsform der Erfindung werden Ester verwendet deren Gesamt C Zahl kleiner gleich 24, bevorzugt kleiner gleich 22 beträgt.

Erfindungsgemäß bevorzugt ist die Verwendung von Estern des 2-Propylheptanols mit Carbonsäuren, die ausgewählt sind aus den C4 bis C30, insbesondere C6 bis C24, insbesondere C6 bis C22, insbesondere C6 bis C18, insbesondere C8 bis C18 bevorzugt C8 bis C16, vorzugsweise C8 bis C12, insbesondere C6 bis C10 Carbonsäuren oder den entsprechenden Dicarbonsäuren.

Erfindungsgemäß eignen sich für die Verwendung Ester des 2-Propylheptanols mit C4 bis C36, C5 bis C30, C6 bis C26, C7 bis C24, C8 bis C22, C9 bis C20, C10 bis C18, C 11 bis C17, C11 bis C16, C12 bis C15, C13 bis C14 Carbonsäuren oder den entsprechenden Dicarbonsäuren.

In einer besonders bevorzugten Ausführungsform der Erfindung werden Estern des 2-Propylheptanols mit Carbonsäuren, die ausgewählt sind aus den C6 bis C12 Carbonsäure sowie Estern des 2-Propylheptanols mit Dicarbonsäuren, die ausgewählt sind aus den C6 bis C12 Dicarbonsäuren, eingesetzt.

Die Verwendung von Estern des 2-Propylheptanols mit gesättigten Carbonsäuren ist erfindungsgemäß bevorzugt.

Die Verwendung von Estern des 2-Propylheptanols mit gesättigten Dicarbonsäuren ist erfindungsgemäß bevorzugt.

Die Verwendung von Estern des 2-Propylheptanols mit linearen, unverzweigten Carbonsäuren ist erfindungsgemäß bevorzugt.

Die Verwendung von Estern des 2-Propylheptanols mit linearen, unverzweigten Dicarbonsäuren ist erfindungsgemäß bevorzugt.

Der Begriff "CX Carbonsäuren" umfasst Carbonsäuren mit einer Gesamtkohlenstoff Anzahl von X, also z.B. "C8 Carbonsäuren" umfasst alle Carbonsäuren, welche eine Gesamt Kohlenstoff Zahl von 8 haben, wie beispielsweise n-Octansäure, Isooctansäuren oder Methylheptansäuren, Entsprechend umfasst der Begriff "CX Dicarbonsäuren" alle Säuren mit 2 Carboxygruppen, welche eine Gesamt C Zahl von X aufweisen, so umfasst z.B. "C4 Dicarbonsäure" u.a. Butandisäure (Bernsteinsäure) sowie Maleinsäure und Fumarsäure.

Der Begriff "Carbonsäure" bezeichnet im Rahmen der vorliegenden Erfindung "Monocarbonsäuren".

Die sensorische Prüfung von 2-Propylheptylcaprylat und 2-Propylheptylcapronat zeigt eine signifikante Verbesserung der Sensorik - insbesondere bezüglich der Spreitung - gegenüber bekannten Emollients (z.B. verschiedene andere Esteröle oder Dialkylcarbonate).

Erfindungsgemäß sind Ester des 2-Propylheptanols mit beispielsweise (in Klammern Trivialnamen der Säuren) n-Butansäure (Buttersäure), 2-Methylpropansäure (Isobuttersäure), Pentansäure (Valeriansäure), i-Pentansäure, wie z.B. 2,2-Dimethylpropansäure (Pivalinsäure, Neopentansäure) und 3-Methylbutansäure (iso-Pentansäure, iso-Valeriansäure), Hexansäure (Capronsäure), Heptansäure, Octansäure (Caprylsäure), i-Octansäure wie z.B. insbesondere 2-Propylheptyl-2-ethylhexansäureester, aber auch 2-Propylheptyl-3-ethylhexansäureester 2-Propylheptyl-4-ethylhexansäureester, 2-Propylheptyl-5-ethylhexansäureester sowie technische Gemische von verzweigten Octansäuren, wie sie beispielsweise unter dem Handelsnamen Cekanoic® C8 von der Fa. Exxon vertrieben werden. Nonansäure (Pelargonsäure, Nonylsäure), Decansäure (Caprinsäure), i-Decansäuren, wie z.B. Trimethylheptansäure (Neodecansäure, Isodecansäure) sowie technische Gemische verzweigter Decansäuren, wie sich beispielsweise unter dem Handelsnamen Cekanoic® C10 von der Fa. Exxon vertrieben werden, Undecansäure, Undecensäure, Dodecansäure (Laurinsäure), Tridecansäure, Tetradecansäure (Myristinsäure), Pentadecansäure, Hexadecansäure (Palmitinsäure), Heptadecansäure(Margarinsäure), Octadecansäure (Stearinsäure), Nonadecansäure, Eicosansäure, Docosansäure, Tetracosansäure, Hexacosanäure, Dimerfettsäuren (C36, wie beispielsweise unter dem Handelsnamen "Empol 1062" von der Fa. Cognis erhältlich) Talkfettsäuren, Kokosfettsäuren, Palmfettsäuren, Rizinolsäure, Ölsäure, Linolsäure, Linolensäure, Isostearinsäure, Isooctansäure, Isononansäure, Isodecansäure, 2-Ethylhexansäure, 2-Propylheptansäure, 2-Butyloctansäure, 2-Butyldecansäure,2-Hexyloctansäure, 2-Hexyldecansäure, 2-Hexyldodecansäure, 2-Octyldecansäure, oder Dicarbonsäuren wie z.B. Fumarsäure, Maleinsäure, Adipinsäure, Pimelinsäure, Korksäure, Azelainsäure, Sebazinsäure. Geeignet sind auch Ester des 2-Propylheptanols mit Cekanoic®C8 (Isooctansäure), Cekanoic®C9 (Isononansäure: 3,5,5-Trimethylhexansäure und 2,5,5-Trimethylhexansäure) und Cekanoic®C10 (Isodecansäure) von der Fa. Exxon Mobile, die Carbonsäure-Isomerengemische darstellen.

Der Begriff "Ester von 2-Propylheptanol mit Dicarbonsäuren" umfasst sowohl Diester der Dicarbonsäuren mit 2-Propylheptanol, also beispielsweise Di-2-Propylheptyl-n-octandisäurediester als auch Monoester, wie z.B. 2-Propyl-heptyl-n-octandisäuremonoester als auch gemischte Ester, in denen eine Säuregruppe der Dicarbonsäure mit 2-Propylheptanol verestert ist und die zweite Säuregruppe der Dicarbonsäure mit einem weiteren Alkohol verestert ist. In einer Ausführungsform der Erfindung werden gemischte Ester von Dicarbonsäuren und 2-Propylheptanol und einem weiteren Alkohol, ausgewählt aus der Gruppe bestehend aus 3-Methyl-2-propylhexanol, 4-Methyl-2-propylhexanol und 5-Methyl-2-propylhexanol eingesetzt. In einer bevorzugten Ausführungsform der Erfindung werden die gemischten Ester erhalten durch Umsetzung der entsprechenden Dicarbonsäure mit einem Gemisch aus 2-Propylheptanol, 3-Methyl-2-propylhexanol, 4-Methyl-2-propylhexanol und 5-Methyl-2-propylhexanol. In einer weiteren Ausführungsform werden eingesetzt gemischte Ester von Dicarbonsäuren und 2-Propylheptanol und einem weiteren Alkohol der allgemeinen Formel R-OH, wobei R für einen linearen oder verzweigten, gesättigten oder ungesättigten Alkylrest mit 1 bis 12 C-Atomen steht.

Ein weiterer Gegenstand der Erfindung sind Ester von 2-Propylheptanol mit Carbonsäuren ausgewählt aus linearen, verzweigten, gesättigten oder ungesättigten C4 bis C36 Carbonsäuren, mit Ausnahme von 2-Propylheptylmethacrylat. Die Erfindung umfasst sowohl einzelne Ester aus auch Mischungen von verschiedenen Estern.

Ein bevorzugter Gegenstand der Erfindung sind Ester von 2-Propylheptanol mit linearen, verzweigten, gesättigten oder ungesättigten C4 bis C30 Carbonsäuren, insbesondere C6 bis C24, insbesondere C6 bis C22, insbesondere C6 bis C18, insbesondere C8 bis C18, insbesondere C8 bis C16 bevorzugt C8 bis C16, vorzugsweise C8 bis C12, insbesondere C6 bis C10 Carbonsäuren.

Erfindungsgemäße sind Ester des 2-Propylheptanols mit C4 bis C36, C5 bis C30, C6 bis C26, C7 bis C24, C8 bis C22, C9 bis C20, C10 bis C18, C 11 bis C17, C11 bis C16, C12 bis C15, C13 bis C14 Carbonsäuren.

Ein bevorzugter Gegenstand der Erfindung sind Ester des2-Propylheptanols mit Carbonsäuren, die ausgewählt sind aus den C6 bis C12 Carbonsäuren.

Ester des 2-Propylheptanols mit gesättigten Carbonsäuren sind erfindungsgemäß bevorzugt.

Ester von 2-Propylheptanol mit linearen, gesättigten Carbonsäuren sind erfindungsgemäß bevorzugt.

Die vorliegende Erfindung umfasst Ester des 2-Propylheptanol mit linearen und/oder verzweigten Carbonsäuren. Eine Ausführungsform der Erfindung betrifft Ester des 2-Propylheptanols mit verzweigten Carbonsäuren: Unter dem Begriff "i-Säure" mit X C-Atomen im Sinne der Erfindung sind alle verzweigten Carbonsäuren zu verstehen, welche in Summe X C-Atome enthalten. So z.B. Methyl-, Ethyl- oder Propylverzweigte, gegebenenfalls mehrfachverzweigte Carbonsäuren. In einer besonderen Ausführungsform wird die Untergruppe der - gegebenenfalls mehrfach - methylverzweigten Carbonsäuren eingesetzt (= Iso-Säuren).

Bevorzugt sind die folgenden Ester 2-Propylheptyl-n-hexansäureester, 2-Propylheptyl-n-heptansäureester, 2-Propylheptyl-n-octansäureester, 2-Propylheptyl-n-nonansäureester, 2-Propylheptyl-n-decansäureester, 2-Propylheptyl-n-undecansäureester, 2-Propylheptyl-n-dodecansäureester.

Besonders bevorzugt sind die folgenden Ester: 2-Propylheptyl-n-hexansäureester, 2-Propylheptyl-n-octansäureester, 2-Propylheptyl-n-decansäureester, 2-Propylheptyl-n-dodecansäureester.

Bevorzugt sind die folgenden Ester 2-Propylheptyl-n-hexansäureester, 2-Propylheptyl-i-hexansäureester, 2-Propylheptyl-n-heptansäureester, 2-Propylheptyl-i-heptansäureester, 2-Propylheptyl-n-octansäureester, 2-Propylheptyl-i-octansäureester, 2-Propylheptyl-n-nonansäureester, 2-Propylheptyl-i-nonansäureester, 2-Propylheptyl-n-decansäureester, 2-Propylheptyl-i-decansäureester, 2-Propylheptyl-n-undecansäureester, 2-Propylheptyl-i-undecansäureester, 2-Propylheptyl-n-dodecansäureester, 2-Propylheptyl-i-dodecansäureester.

Besonders bevorzugt sind die folgenden Ester 2-Propylheptyl-n-hexansäureester, 2_Propylheptyl-i-hexansäureester, 2-Propylheptyl-n-octansäureester, 2-Propylheptyl-i-octansäureester, 2-Propylheptyl-n-decansäureester, 2-Propylheptyl-i-decansäureester, 2-Propylheptyl-n-dodecansäureester, 2-Propylheptyl-i-dodecansäureester.

Gegenstand der Erfindung sind Zusammensetzungen, enthaltend einen Ester von 2-Propylheptanol mit einer C4 bis C36 Carbonsäure oder einer C4 bis C36 Dicarbonsäure und mindestens einen weiteren Ester derselben Säure mit einem Alkohol ausgewählt aus der Gruppe bestehend aus 3-Methyl-2-Propylhexanol, 4-Methyl-2-Propylhexanol oder 5-Methyl-2-Propylhexanol.

In einer bevorzugten Ausführungsform der Erfindung enthaltend diese Zusammensetzungen
- 80 bis 99,99 Gew.-% des 2-Propylheptylesters
- 0,01 bis 20 Gew.-% der entsprechenden Methyl-2-Propylhexylester.

Ein Gegenstand der Erfindung betrifft 2-Propylheptyl-n-butansäureester.
Ein Gegenstand der Erfindung betrifft Zusammensetzungen, enthaltend 2-Propylheptyl-n-butansäureester und mindestens einen weiteren Ester ausgewählt aus der Gruppe bestehend aus 3-Methyl-2-propylhexyl-n-butansäureester, 4-Methyl-2-propylhexyl-n-butansäureester, 5-Methyl-2-propylhexyl-n-butansäureester und Mischungen daraus.

Ein Gegenstand der Erfindung betrifft 2-Propylheptyl-i-butansäureester.

Ein Gegenstand der Erfindung betrifft Zusammensetzungen, enthaltend 2-Propylheptyl-i-butansäureester und mindestens einen weiteren Ester ausgewählt aus der Gruppe bestehend aus 3-Methyl-2-propylhexyl-i-butansäureester, 4-Methyl-2-propylhexyl-i-butansäureester, 5-Methyl-2-propylhexyl-i-butansäureester und Mischungen daraus.

Ein Gegenstand der Erfindung betrifft 2-Propylheptyl-n-pentansäureester.

Ein Gegenstand der Erfindung betrifft Zusammensetzungen, enthaltend 2-Propylheptyl-n-pentansäureester und mindestens einen weiteren Ester ausgewählt aus der Gruppe bestehend aus 3-Methyl-2-propylhexyl-n-pentansäureester, 4-Methyl-2-propylhexyl-n-pentansäureester, 5-Methyl-2-propylhexyl-n-pentansäureester und Mischungen daraus.

Ein Gegenstand der Erfindung betrifft 2-Propylheptyl-i-pentansäureester.

Ein Gegenstand der Erfindung betrifft Zusammensetzungen, enthaltend 2-Propylheptyl-i-pentansäureesterund mindestens einen weiteren Ester ausgewählt aus der Gruppe bestehend aus 3-Methyl-2-propylhexyl-i-pentansäureester, 4-Methyl-2-propylhexyl-i-pentansäureester, 5-Methyl-2-propylhexyl-i-pentansäureester und Mischungen daraus.

Ein Gegenstand der Erfindung betrifft 2-Propylheptyl-n-hexansäureester.
Ein Gegenstand der Erfindung betrifft Zusammensetzungen, enthaltend 2-Propylheptyl-n-hexansäureester und mindestens einen weiteren Ester ausgewählt aus der Gruppe bestehend aus 3-Methyl-2-propylhexyl-n-hexansäureester, 4-Methyl-2-propylhexyl-n-hexansäureester, 5-Methyl-2-propylhexyl-n-hexansäureester und Mischungen daraus.

Ein Gegenstand der Erfindung betrifft 2-Propylheptyl-i-hexansäureester.
Ein Gegenstand der Erfindung betrifft Zusammensetzungen, enthaltend 2-Propylheptyl-i-hexansäureester und mindestens einen weiteren Ester ausgewählt aus der Gruppe bestehend aus 3-Methyl-2-propylhexyl-i-hexansäureester, 4-Methyl-2-propylhexyl-i-hexansäureester, 5-Methyl-2-propylhexyl-i-hexansäureester und Mischungen daraus.

Ein Gegenstand der Erfindung betrifft 2- Propylheptyl-n-heptansäureester.
Ein Gegenstand der Erfindung betrifft Zusammensetzungen, enthaltend 2-Propylheptyl-n-heptansäureester und mindestens einen weiteren Ester ausgewählt aus der Gruppe bestehend aus 3-Methyl-2-propylhexyl-n-heptansäureester, 4-Methyl-2-propylhexyl-n-heptansäureester, 5-Methyl-2-propylhexyl-n-heptansäureester und Mischungen daraus.

Ein Gegenstand der Erfindung betrifft 2- Propylheptyl-i-heptansäureester.
Ein Gegenstand der Erfindung betrifft Zusammensetzungen, enthaltend 2-Propylheptyl-i-heptansäureester und mindestens einen weiteren Ester ausgewählt aus der Gruppe bestehend aus 3-Methyl-2-propylhexyl-i-heptansäureester, 4-Methyl-2-propylhexyl-i-heptansäureester, 5-Methyl-2-propylhexyl-i-heptansäureester und Mischungen daraus.

Ein Gegenstand der Erfindung betrifft 2-Propylheptyl-n-octansäureester.
Ein Gegenstand der Erfindung betrifft Zusammensetzungen, enthaltend 2-Propylheptyl-n-octansäureester und mindestens einen weiteren Ester ausgewählt aus der Gruppe bestehend aus 3-Methyl-2-propylhexyl-n-octansäureester, 4-Methyl-2-propylhexyl-n-octansäureester, 5-Methyl-2-propylhexyl-n-octansäureester und Mischungen daraus.

Ein Gegenstand der Erfindung betrifft 2-Propylheptyl-i-octansäureester.
Zusammensetzungen, enthaltend 2-Propylheptyl-i-octansäureester und mindestens einen weiteren Ester ausgewählt aus der Gruppe bestehend aus 3-Methyl-2-propylhexyl-i-octansäureester, 4-Methyl-2-propylhexyl-i-octansäureester, 5-Methyl-2-propylhexyl-i-octansäureester und Mischungen daraus

Ein Gegenstand der Erfindung betrifft 2- Propylheptyl-n-nonansäureester.
Ein Gegenstand der Erfindung betrifft Zusammensetzungen, enthaltend 2-Propylheptyl-n-nonansäureester und mindestens einen weiteren Ester ausgewählt aus der Gruppe bestehend aus 3-Methyl-2-propylhexyl-n-nonansäureester, 4-Methyl-2-propylhexyl-n-nonansäureester, 5-Methyl-2-propylhexyl-n-nonansäureester und Mischungen daraus.

Ein Gegenstand der Erfindung betrifft 2- Propylheptyl-i-nonansäureester.
Ein Gegenstand der Erfindung betrifft Zusammensetzungen, enthaltend 2-Propylheptyl-i-nonansäureester und mindestens einen weiteren Ester ausgewählt aus der Gruppe bestehend aus 3-Methyl-2-propylhexyl-i-nonansäureester, 4-Methyl-2-propylhexyl-i-nonansäureester, 5-Methyl-2-propylhexyl-i-nonansäureester und Mischungen daraus.

Ein Gegenstand der Erfindung betrifft 2-Propylheptyl-n-decansäureester.
Ein Gegenstand der Erfindung betrifft Zusammensetzungen, enthaltend 2-Propylheptyl-n-decansäureester und mindestens einen weiteren Ester ausgewählt aus der Gruppe bestehend aus 3-Methyl-2-propylhexyl-n-decansäureester, 4-Methyl-2-propylhexyl-n-decansäureester, 5-Methyl-2-propylhexyl-n-decansäureester und Mischungen daraus.

Ein Gegenstand der Erfindung betrifft 2-Propylheptyl-i-decansäureester.
Ein Gegenstand der Erfindung betrifft Zusammensetzungen, enthaltend 2-Propylheptyl-i-decansäureester und mindestens einen weiteren Ester ausgewählt aus der Gruppe bestehend aus 3-Methyl-2-propylhexyl-i-decansäureester, 4-Methyl-2-propylhexyl-i-decansäureester, 5-Methyl-2-propylhexyl-i-decansäureester und Mischungen daraus.

Ein Gegenstand der Erfindung betrifft 2-Propylheptyl-n-undecansäureester.
Ein Gegenstand der Erfindung betrifft Zusammensetzungen, enthaltend 2-Propylheptyl-n-undecansäureester und mindestens einen weiteren Ester ausgewählt aus der Gruppe bestehend aus 3-Methyl-2-propylhexyl-n-undecansäureester, 4-Methyl-2-propylhexyl-n-undecansäureester, 5-Methyl-2-propylhexyl-n-undecansäureester und Mischungen daraus.

Ein Gegenstand der Erfindung betrifft 2-Propylheptyl-i-undecansäureester.
Ein Gegenstand der Erfindung betrifft Zusammensetzungen, enthaltend 2-Propylheptyl-i-undecansäureester und mindestens einen weiteren Ester ausgewählt aus der Gruppe bestehend aus 3-Methyl-2-propylhexyl-i-undecansäureester, 4-Methyl-2-propylhexyl-i-undecansäureester, 5-Methyl-2-propylhexyl-i-undecansäureester und Mischungen daraus.

Ein Gegenstand der Erfindung betrifft 2-Propylheptyl-n-undecensäureester.
Ein Gegenstand der Erfindung betrifft Zusammensetzungen, enthaltend 2-Propylheptyl-n-undecensäureester und mindestens einen weiteren Ester ausgewählt aus der Gruppe bestehend aus 3-Methyl-2-propylhexyl-n-undecensäureester, 4-Methyl-2-propylhexyl-n-undecensäureester, 5-Methyl-2-propylhexyl-n-undecensäureester und Mischungen daraus.

Ein Gegenstand der Erfindung betrifft 2-Propylheptyl-n-dodecansäureester.
Ein Gegenstand der Erfindung betrifft Zusammensetzungen, enthaltend 2-Propylheptyl-n-dodecansäureester und mindestens einen weiteren Ester ausgewählt aus der Gruppe bestehend aus 3-Methyl-2-propylhexyl-n-dodecansäureester, -Methyl-2-propylhexyl-n-dodecansäureester, 5-Methyl-2-propylhexyl-n-dodecansäureester und Mischungen daraus.

Ein Gegenstand der Erfindung betrifft 2-Propylheptyl-i-dodecansäureester.
Ein Gegenstand der Erfindung betrifft Zusammensetzungen, enthaltend 2-Propylheptyl-i-dodecansäureester und mindestens einen weiteren Ester ausgewählt aus der Gruppe bestehend aus 3-Methyl-2-propylhexyl-i-dodecansäureester, 4-Methyl-2-propylhexyl-i-dodecansäureester, 5-Methyl-2-propylhexyl-i-dodecansäureester und Mischungen daraus.

Überraschenderweise hat sich gezeigt, dass sich die Ester bzw. Zusammensetzungen nach mindestens einem der Ansprüche 2-10 besonders eignen für die Verwendung in kosmetischen und/oder pharmazeutischen Zubereitungen. Ein weiterer Gegenstand der Erfindung betrifft daher die Verwendung eines Esters bzw. einer Zusammensetzungen nach mindestens einem der Ansprüche 2-10 in kosmetischen und/oder pharmazeutischen Zubereitungen.

Ein Gegenstand der Erfindung betrifft 2-Propylheptyl-n-tridecansäureester.
Ein Gegenstand der Erfindung betrifft Zusammensetzungen, enthaltend 2-Propylheptyl-n-tridecansäureester und mindestens einen weiteren Ester ausgewählt aus der Gruppe bestehend aus 3-Methyl-2-propylhexyl-n-tridecansäureester, 4-Methyl-2-propylhexyl-n-tridecansäureester, 5-Methyl-2-propylhexyl-n-tridecansäureester und Mischungen daraus.

Ein Gegenstand der Erfindung betrifft 2-Propylheptyl-i-tridecansäureester.
Ein Gegenstand der Erfindung betrifft Zusammensetzungen, enthaltend 2-Propylheptyl-i-tridecansäureester und mindestens einen weiteren Ester ausgewählt aus der Gruppe bestehend aus 3-Methyl-2-propylhexyl-i-tridecansäureester, 4-Methyl-2-propylhexyl-i-tridecansäureester, 5-Methyl-2-propylhexyl-i-tridecansäureester und Mischungen daraus.

Ein Gegenstand der Erfindung betrifft 2-Propylheptyl-n-tetradecansäureester.
Ein Gegenstand der Erfindung betrifft Zusammensetzungen, enthaltend 2-Propylheptyl-n-tetradecansäureester und mindestens einen weiteren Ester ausgewählt aus der Gruppe bestehend aus 3-Methyl-2-propylhexyl-n-tetradecansäureester, 4-Methyl-2-propylhexyl-n-tetradecansäureester, 5-Methyl-2-propylhexyl-n-tetradecansäureester und Mischungen daraus.

Ein Gegenstand der Erfindung betrifft 2-Propylheptyl-i-tetradecansäureester.
Ein Gegenstand der Erfindung betrifft Zusammensetzungen, enthaltend 2-Propylheptyl-i-tetradecansäureester und mindestens einen weiteren Ester ausgewählt aus der Gruppe bestehend aus 3-Methyl-2-propylhexyl-i-tetradecansäureester, 4-Methyl-2-propylhexyl-i-tetradecansäureester, 5-Methyl-2-propylhexyl-i-tetradecansäureester und Mischungen daraus.

Ein Gegenstand der Erfindung betrifft 2-Propylheptyl-n-pentadecansäureester.
Ein Gegenstand der Erfindung betrifft Zusammensetzungen, enthaltend 2-Propylheptyl-n-pentadecansäureester und mindestens einen weiteren Ester ausgewählt aus der Gruppe bestehend aus 3-Methyl-2-propylhexyl-n-pentadecansäureester, 4-Methyl-2-propylhexyl-n-pentadecansäureester, 5-Methyl-2-propylhexyl-n-pentadecansäureester und Mischungen daraus.

Ein Gegenstand der Erfindung betrifft 2-Propylheptyl-i-pentadecansäureester.
Ein Gegenstand der Erfindung betrifft Zusammensetzungen, enthaltend 2-Propylheptyl-i-pentadecansäureester und mindestens einen weiteren Ester ausgewählt aus der Gruppe bestehend aus 3-Methyl-2-propylhexyl-i-pentadecansäureester, 4-Methyl-2-propylhexyl-i-pentadecansäureester, 5-Methyl-2-propylhexyl-i-pentadecansäureester und Mischungen daraus.

Ein Gegenstand der Erfindung betrifft 2-Propylheptyl-n-hexadecansäureester.
Ein Gegenstand der Erfindung betrifft Zusammensetzungen, enthaltend 2-Propylheptyl-n-hexadecansäureester und mindestens einen weiteren Ester ausgewählt aus der Gruppe bestehend aus 3-Methyl-2-propylhexyl-n-hexadecansäureester, 4-Methyl-2-propylhexyl-n-hexadecansäureester, 5-Methyl-2-propylhexyl-n-hexadecansäureester und Mischungen daraus.

Ein Gegenstand der Erfindung betrifft 2-Propylheptyl-i-hexadecansäureester.
Ein Gegenstand der Erfindung betrifft Zusammensetzungen, enthaltend 2-Propylheptyl-i-hexadecansäureester und mindestens einen weiteren Ester ausgewählt aus der Gruppe bestehend aus 3-Methyl-2-propylhexyl-i-hexadecansäureester, 4-Methyl-2-propylhexyl-i-hexadecansäureester, 5-Methyl-2-propylhexyl-i-hexadecansäureester und Mischungen daraus.

Ein Gegenstand der Erfindung betrifft 2-Propylheptyl-n-heptadecansäureester.
Ein Gegenstand der Erfindung betrifft Zusammensetzungen, enthaltend 2-Propylheptyl-n-heptadecansäureester und mindestens einen weiteren Ester ausgewählt aus der Gruppe bestehend aus 3-Methyl-2-propylhexyl-n-heptadecansäureester, 4-Methyl-2-propylhexyl-n-heptadecansäureester, 5-Methyl-2-propylhexyl-n-heptadecansäureester und Mischungen daraus.

Ein Gegenstand der Erfindung betrifft 2-Propylheptyl-i-heptadecansäureester.
Ein Gegenstand der Erfindung betrifft Zusammensetzungen, enthaltend 2-Propylheptyl-i-heptadecansäureester und mindestens einen weiteren Ester ausgewählt aus der Gruppe bestehend aus 3-Methyl-2-propylhexyl-i-heptadecansäureester, 4-Methyl-2-propylhexyl-i-heptadecansäureester, 5-Methyl-2-propylhexyl-i-heptadecansäureester und Mischungen daraus.

Ein Gegenstand der Erfindung betrifft 2-Propylheptyl-n-octadecansäureester.
Ein Gegenstand der Erfindung betrifft Zusammensetzungen, enthaltend 2-Propylheptyl-n-octadecansäureester und mindestens einen weiteren Ester ausgewählt aus der Gruppe bestehend aus 3-Methyl-2-propylhexyl-n-octadecansäureester, 4-Methyl-2-propylhexyl-n-octadecansäureester, 5-Methyl-2-propylhexyl-n-octadecansäureester und Mischungen daraus.

Ein Gegenstand der Erfindung betrifft 2-Propylheptyl-i-octadecansäureester.
Ein Gegenstand der Erfindung betrifft Zusammensetzungen, enthaltend 2-Propylheptyl-i-octadecansäureester und mindestens einen weiteren Ester ausgewählt aus der Gruppe bestehend aus 3-Methyl-2-propylhexyl-i-octadecansäureester, 4-Methyl-2-propylhexyl-i-octadecansäureester, 5-Methyl-2-propylhexyl-i-octadecansäureesterund Mischungen daraus.

Ein Gegenstand der Erfindung betrifft 2-Propylheptyl-cis-9-octadecensäureester. (= Ester der Ölsäure). Ein Gegenstand der Erfindung betrifft Zusammensetzungen, enthaltend 2-Propylheptyl-cis-9-octadecensäureester und mindestens einen weiteren Ester ausgewählt aus der Gruppe bestehend aus 3-Methyl-2-propylhexyl-cis-9-octadecensäureester, 4-Methyl-2-propylhexyl-cis-9-octadecensäureester, 5-Methyl-2-propylhexyl-cis-9-octadecensäureester und Mischungen daraus.

Ein Gegenstand der Erfindung betrifft 2-Propylheptyl-(Z,Z)-9,12-octadecadiensäureester. (= Ester der Linolsäure). Ein Gegenstand der Erfindung betrifft Zusammensetzungen, enthaltend 2-Propylheptyl-(Z,Z)-9,12-octadecadiensäureester und mindestens einen weiteren Ester ausgewählt aus der Gruppe bestehend aus 3-Methyl-2-propylhexyl-(*Z,Z*)-9,12-octadecadiensäureester, 4-Methyl-2-propylhexyl-(*Z,Z*)-9,12-octadecadiensäureester, 5-Methyl-2-propylhexyl-(*Z,Z*)-9,12-octadecadiensäureester und Mischungen daraus.

Ein Gegenstand der Erfindung betrifft 2-Propylheptyl-(*all-Z*)-9,12,15-octadecatriensäureester. (= Ester der Linolensäure). Ein Gegenstand der Erfindung betrifft Zusammensetzungen, enthaltend 2-Propylheptyl-(*all-Z*)-9,12,15-octadecatriensäureester und mindestens einen weiteren Ester ausgewählt aus der Gruppe bestehend aus 3-Methyl-2-propylhexyl (*all-Z*)-9,12,15-octadecatriensäureester, 4-Methyl-2-propylhexyl-(*all-Z*)-9,12,15-octadecatriensäureester, 5-Methyl-2-propylhexyl-(*all-Z*)-9, 12,15-octadecatriensäureester und Mischungen daraus.

Ein Gegenstand der Erfindung betrifft 2-Propylheptyl-n-nonadecansäureester.
Ein Gegenstand der Erfindung betrifft Zusammensetzungen, enthaltend 2-Propylheptyl-n-nonadecansäureester und mindestens einen weiteren Ester ausgewählt aus der Gruppe bestehend aus 3-Methyl-2-propylhexyl-n-nonadecansäureester, 4-Methyl-2-propylhexyl-n-nonadecansäureester, 5-Methyl-2-propylhexyl-n-nonadecansäureester und Mischungen daraus.

Ein Gegenstand der Erfindung betrifft 2-Propylheptyl-i-nonadecansäureester.
Ein Gegenstand der Erfindung betrifft Zusammensetzungen, enthaltend 2-Propylheptyl-i-nonadecansäureester und mindestens einen weiteren Ester ausgewählt aus der Gruppe bestehend aus 3-Methyl-2-propylhexyl-i-nonadecansäureester, 4-Methyl-2-propylhexyl-i-nonadecansäureester, 5-Methyl-2-propylhexyl-i-nonadecansäureester und Mischungen daraus.

Ein Gegenstand der Erfindung betrifft 2-Propylheptyl-n-eicosansäureester.
Ein Gegenstand der Erfindung betrifft Zusammensetzungen, enthaltend 2-Propylheptyl-n-eicosansäureester und mindestens einen weiteren Ester ausgewählt aus der Gruppe bestehend aus 3-Methyl-2-propylhexyl-n-eicosansäureester, 4-Methyl-2-propylhexyl-n-eicosansäureester, 5-Methyl-2-propylhexyl-n-eicosansäureester und Mischungen daraus.

Ein Gegenstand der Erfindung betrifft 2-Propylheptyl-i-eicosansäureester.
Ein Gegenstand der Erfindung betrifft Zusammensetzungen, enthaltend 2-Propylheptyl-i-eicosansäureester und mindestens einen weiteren Ester ausgewählt aus der Gruppe bestehend aus 3-Methyl-2-propylhexyl-i-eicosansäureester, 4-Methyl-2-propylhexyl-i-eicosansäureester, 5-Methyl-2-propylhexyl-i-eicosansäureester und Mischungen daraus.

Ein Gegenstand der Erfindung betrifft 2-Propylheptyl-n-docosansäureester. -
Ein Gegenstand der Erfindung betrifft Zusammensetzungen, enthaltend 2-Propylheptyl-n-docosansäureester und mindestens einen weiteren Ester ausgewählt aus der Gruppe bestehend aus 3-Methyl-2-propylhexyl-n-docosansäureester, 4-Methyl-2-propylhexyl-n-docosansäureester, 5-Methyl-2-propylhexyl-n-docosansäureester und Mischungen daraus.

Ein Gegenstand der Erfindung betrifft 2-Propylheptyl-i-docosansäureester.
Ein Gegenstand der Erfindung betrifft Zusammensetzungen, enthaltend 2-Propylheptyl-i-docosansäureester und mindestens einen weiteren Ester ausgewählt aus der Gruppe bestehend aus 3-Methyl-2-propylhexyl-i-docosansäureester, 4-Methyl-2-propylhexyl-i-docosansäureester, 5-Methyl-2-propylhexyl-i-docosansäureester und Mischungen daraus.

Ein Gegenstand der Erfindung betrifft 2-Propylheptyl-n-tetracosansäureester.
Ein Gegenstand der Erfindung betrifft Zusammensetzungen, enthaltend 2-Propylheptyl-n-tetracosansäureester und mindestens einen weiteren Ester ausgewählt aus der Gruppe bestehend aus 3-Methyl-2-propylhexyl-n-tetracosansäureester, 4-Methyl-2-propylhexyl-n-tetracosansäureester, 5-Methyl-2-propylhexyl-n-tetracosansäureester und Mischungen daraus.

Ein Gegenstand der Erfindung betrifft 2-Propylheptyl-i-tetracosansäureester.
Ein Gegenstand der Erfindung betrifft Zusammensetzungen, enthaltend 2-Propylheptyl-i-tetracosansäureester und mindestens einen weiteren Ester ausgewählt aus der Gruppe bestehend aus 3-Methyl-2-propylhexyl-i-tetracosansäureester, 4-Methyl-2-propylhexyl-i-tetracosansäureester, 5-Methyl-2-propylhexyl-i-tetracosansäureesterund Mischungen daraus.

Ein Gegenstand der Erfindung betrifft 2-Propylheptyl-n-hexacosansäureester.
Ein Gegenstand der Erfindung betrifft Zusammensetzungen, enthaltend 2-Propylheptyl-n-hexacosansäureester und mindestens einen weiteren Ester ausgewählt aus der Gruppe bestehend aus 3-Methyl-2-propylhexyl-n-hexacosansäureester, 4-Methyl-2-propylhexyl-n-hexacosansäureester, 5-Methyl-2-propylhexyl-n-hexacosansäureester und Mischungen daraus.

Ein Gegenstand der Erfindung betrifft 2-Propylheptyl-i-hexacosansäureester.
Ein Gegenstand der Erfindung betrifft Zusammensetzungen, enthaltend 2-Propylheptyl-i-hexacosansäureester und mindestens einen weiteren Ester ausgewählt aus der Gruppe bestehend aus 3-Methyl-2-propylhexyl-i-hexacosansäureester, 4-Methyl-2-propylhexyl-i-hexacosansäureester, 5-Methyl-2-propylhexyl-i-hexacosansäureester und Mischungen daraus.

Gegenstand ist auch ein Verfahren zur Herstellung der erfindungsgemäßen Ester, wobei man eine Mischung enthaltend 2-Propylheptanol und die entsprechende Säure umsetzt.

Gegenstand der Erfindung ist demnach ein Verfahren zur Herstellung der Ester gemäß dem Anspruch 3 wobei man eine Mischung enthaltend 2-Propylheptanol und die entsprechende Säure (n-Butansäure, i-Butansäure, n-Pentansäure, i-Pentansäure, n-Hexansäure, i-Hexansäure, n-Heptansäure, i- Heptansäure, n-Octansäure, i-Octansäure, n-Nonansäure, i-Nonansäure, n-Decansäure, i-Decansäure, n-Undecansäure, i-Undecansäure, n-Undecensäure, n-Dodecansäure oder i-Dodecansäure) umsetzt.

Das erfindungsgemäße Verfahren umfasst ebenfalls die Herstellung von Estergemischen, bei dem man2-Propylheptanol zusammen mit den entsprechenden Säuregemischen umsetzt.

In einer bevorzugten Ausführungsform der Erfindung setzt man die Mischung enthaltend Alkohol und die entsprechende Säure unter Zusatz eines Veresterungskatalysators um.

In einer bevorzugten Ausführungsform wird die Mischung enthaltend Alkohol und die entsprechende Säure erhitzt, das entstehende Wasser kontinuierlich abgeführt und das Rohprodukt danach destilliert. Das Verfahren kann unter Zusatz eines Veresterungskatalysators, z.B. säurekatalytisch oder basenkatalytisch durchgeführt werden. In einer bevorzugten Ausführungsform wird das Verfahren ohne Zusatz von Lösungsmitteln durchgeführt, vorzugsweise mit Edukten, die möglichst wasserfrei sind. In einer bevorzugten Ausführungsform des Verfahrens wird ein Zinn-Katalysator einsetzt. Als Zinn-Katalysatoren geeignet sind beispielsweise Zinnoxalat (z. B. Fascat® 2001), Zinnoxid (SnO, Fascat® 2000) sowie Zinn-IV-Katalysatoren wie Dibutylzinndiacetat Fascat® 4200), Dibutlyzinnoxid (Fascat® 4201), und Dibutylzinnlaurat (Fascat® 4202) oder Zinnoxid (SnO), die ehemals von Atofina und gegenwärtig von Arkema vermarktet werden.

Vorzugsweise wird die Veresterung bei Temperaturen zwischen 100- 300 °C, insbesondere 200 - 250 °C durchführt.

In einer weiteren Ausführungsform wird als Katalysator mindestens ein Enzym eingesetzt. Als Enzym eignen sich alle dem Fachmann bekannten Enzyme oder Enzymmischungen, welche in der Lage sind die Veresterung von Alkohol und Säure zu katalysieren, exemplarisch seinen genannt Lipasen, Acyltransferasen und Esterasen. Die enzymatisch katalysierte Veresterung wird üblicherweise bei Temperaturen von 20 bis 100 °C, vorzugsweise 40 bis 80 °C durchgeführt.

Gegenstand der Erfindung ist ein Verfahren zur Herstellung der Ester gemäß dem Anspruch 3 wobei man eine Mischung enthaltend 2-Propylheptanol und den Methylester der entsprechenden Säure (n-Bufansäure-methylester, i-Butansäure-methylester, n-Pentansäure-methylester, i-Pentansäure-methylester, n-Hexansäure-methylester, i-Hexansäure-methylester, n-Heptan-methylester, 1-Heptansäure-methylester, n-Octansäure-methylester, i-Octansäure-methylester, n-Nonansäure-methylester, i-Nonansäure-methylester, n-Decansäure-methylester, 1-Decansäure-methylester, n-Undecansäure-methylester, i-Undecansäure-methylester, n-Undecensäuremethylester, n-Dodecansäure-methylester, i-Docecansäure-methylester) unter Zusatz eines Umesterungskatalysators umsetzt.

Das erfindungsgemäße Verfahren umfasst ebenfalls die Herstellung von Estergemischen, bei dem man 2-Propylheptanol zusammen mit den entsprechenden Mischungen der Methylestern der Säuren unter Zusatz eines Umesterungskatalysators umsetzt.

In einer bevorzugten Ausführungsform wird die Mischung enthaltend Alkohol und den Methylesterder entsprechende Säure unter Zusatz des Veresterungskatalysators erhitzt, das entstehende Wasser kontinuierlich abgeführt und das Rohprodukt danach destilliert. In einer bevorzugten Ausführungsform wird das Verfahren ohne Zusatz von Lösungsmitteln durchgeführt, vorzugsweise mit Edukten, die möglichst wasserfrei sind.

Vorzugsweise wird die Veresterung bei Temperaturen zwischen 100- 300 °C, insbesondere 200-250°C durchführt. Als Umesterungskatalysator können alle dem Fachmann bekannten Umesterungskatalysatoren eingesetzt, werden, vorzugsweise wird als Umesterungskatalysator Natriummethylat oder Tetraalkyltitanat eingesetzt.

In einer weiteren Ausführungsform wird als Katalysator mindestens ein Enzym eingesetzt. Als Enzym eignen sich alle dem Fachmann bekannten Enzyme oder Enzymmischungen, welche in der Lage sind die Umesterung von Alkohol und Säuremethylester zu katalysieren, exemplarisch seinen genannt Lipasen, Acyltransferasen und Esterasen. Die enzymatisch katalysierte Veresterung wird üblicherweise bei Temperaturen von 20 bis 100 °C, vorzugsweise 40 bis 80 °C durchgeführt.

### Ester von 3-Methyl-2-propylhexanol mit C4 bis C36 Carbonsäuren

Ein weiterer Gegenstand der Erfindung betrifft Ester von 3-Methyl-2-propylhexanol mit linearen, verzweigten, gesättigten oder ungesättigten C4 bis C 36 Carbonsäuren, insbesondere mit C4 bis C18, insbesondere mit C6 bis C12 Carbonsäuren. Besonders bevorzugt sind Ester von 3-Methyl-2-propylhexanol mit linearen, gesättigten Carbonsäuren.

Gegenstand der Erfindung sind insbesondere 3-Methyl-2-propylhexyl-n-butansäureester, 3-Methyl-2-propylhexyl-i-butansäureester, 3-Methyl-2-propylhexyl-n-pentansäureester, 3-Methyl-2-propylhexyl-i-pentansäureester, 3-Methyl-2-propylhexyl-n-hexansäureester, 3-Methyl-2-propylhexyl-i-hexansäureester, 3-Methyl-2-propylhexyl-n-heptansäureester, 3-Methyl-2-propylhexyl-i-heptansäureester, 3-Methyl-2-propylhexyl-n-octansäureester, 3-Methyl-2-propylhexyl-i-octansäureester, 3-Methyl-2-propylhexyl-n-nonansäureester, 3-Methyl-2-propylhexyl-i-nonansäureester, 3-Methyl-2-propylhexyl-n-decansäureester, 3-Methyl-2-propylhexyl-i-decansäureester, 3-Methyl-2-propylhexyl-n-undecansäureester, 3-Methyl-2-propylhexyl-i-undecansäureester, 3-Methyl-2-propylhexyl-n-undecensäureester, 3-Methyl-2-propylhexyl-n-dodecansäureester, 3-Methyl-2-propylhexyl-i-dodecansäureester.

Ein weiterer Gegenstand der Erfindung betrifft die Verwendung von Ester von 3-Methyl-2-propylhexanol mit linearen, verzweigten, gesättigten oder ungesättigten Carbonsäuren mit einer 4 bis 36 Kohlenstoffatomen, insbesondere mit 4 bis 18 Kohlenstoffatomen, insbesondere mit 6 bis 12 Kohlenstoffatomen in kosmetischen und/oder pharmazeutischen Zubereitungen. Besonders bevorzugt sind Ester von 3-Methyl-2-propylhexanol mit linearen, gesättigten Carbonsäuren

Die im folgenden verwendeten Begriffe "Ester von X-Methyl-2-propylhexanol mit Dicarbonsäuren" umfassen sowohl Diester der Dicarbonsäuren mit dem jeweiligen Methyl-2-Propylhexanol, also beispielsweise Di-3-Methyl-2-Propylhexyl-n-octandisäurediester oder Di-5-Methyl-2-Propylhexyl-n-octandisäurediester als auch Monoester, wie z.B. 3-Methyl-2-Propylheptyl-n-octandisäuremonoester als auch gemischte Ester, in denen eine Säuregruppe der Dicarbonsäure mit dem jeweiligen Methyl-2-Propylhexanol (= erster Methyl-2-Propylhexanol) verestert ist und die zweite Säuregruppe der Dicarbonsäure mit einem zweiten Alkohol verestert ist. Der zweite Alkohol kann ausgewählt werden aus 3-Methyl-2-propylhexanol, 4-Methyl-2-propylhexanol und 5-Methyl-2-propylhexanol, wobei dieser zweite Alkohol von dem ersten Methyl-2-Propylhexanol verschieden sein muss.

In einerweiteren Ausführungsform werden eingesetzt gemischte Ester von Dicarbonsäuren und dem jeweiligen Methyl-2-Propylhexanol und einem weiteren Alkohol der allgemeinen Formel R-OH, wobei R für einen gesättigten oder ungesättigten, linearen oder verzweigten, Alkylrest mit 1 bis 12 C-Atomen steht.

In einerweiteren Ausführungsform werden eingesetzt gemischte Ester von Dicarbonsäuren und dem jeweiligen Methyl-2-Propylhexanol und einem weiteren Alkohol der allgemeinen Formel R-OH, wobei R für einen gesättigten, linearen oder verzweigten, Alkylrest mit 1 bis 12 C-Atomen steht.

In einer weiteren Ausführungsform der gemischten Ester wird der zweite Alkohol ausgewählt aus der Gruppe die gebildet wird von Methanol, Ethanol, Propanol, Isopropanol Butanol, Isobutanol, Pentanol, Hexanol, Isohexanol, Octanol, Decanol und Dodecanol.

### Kosmetische/pharmazeutische Zubereitungen

Die 2-Propylheptylester erlauben die Herstellung stabiler kosmetischer und pharmazeutischer Emulsionen mit besonders leichtem Hautgefühl.

Ein weiterer Gegenstand der vorliegenden Erfindung sind daher kosmetische und/oder pharmazeutische Zubereitungen enthaltend
(a) wenigstens einen Ester des 2-Propylheptanols mit linearen oder verzweigten, gesättigten oder ungesättigten, C4-C36-Carbonsäuren - oder C4-C36-Dicarbonsäuren, vorzugsweise wenigstens einen Ester des 2-Propylheptanols mit linearen oder verzweigten, gesättigten oder ungesättigten C4-C18-Carbonsäuren - oderC4-C18-Dicarbonsäuren
(b) wenigstens einen Emulgator und/oder Tensid und/oder Wachskomponente und/oder Polymer und/oder einen weiteren Ölkörper.

Ein Gegenstand der vorliegenden Erfindung sind kosmetische und/oder pharmazeutische Zubereitungen enthaltend
a) wenigstens einen Ester des 2-Propylheptanols mit linearen oder verzweigten, gesättigten oder ungesättigten C4-C36-Carbonsäuren - oder C4-C36-Dicarbonsäuren, vorzugsweise wenigstens einen Ester des 2-Propylheptanols mit linearen oder verzweigten, gesättigten oder ungesättigten C4-C18-Carbonsäuren - oder C4-C18-Dicarbonsäuren

### b-1) wenigstens einen Emulgator

Ein Gegenstand der vorliegenden Erfindung sind kosmetische und/oder pharmazeutische Zubereitungen enthaltend
a) wenigstens einen Ester des 2-Propylheptanols mit linearen oder verzweigten, gesättigten oder ungesättigten C4-C36-Carbonsäuren - oder C4-C36-Dicarbonsäuren, vorzugsweise wenigstens einen Ester des 2-Propylheptanols mit linearen oder verzweigten, gesättigten oder ungesättigten C4-C18-Carbonsäuren - oder C4-C18-Dicarbonsäuren

### b-2) wenigstens ein Tensid.

Ein Gegenstand der vorliegenden Erfindung sind kosmetische und/oder pharmazeutische Zubereitungen enthaltend
a) wenigstens einen Ester des 2-Propylheptanols mit linearen oder verzweigten, gesättigten oder ungesättigten C4-C36-Carbonsäuren - oder C4-C36-Dicarbonsäuren, vorzugsweise wenigstens einen Ester des 2-Propylheptanols mit linearen oder verzweigten, gesättigten oder ungesättigten C4-C18-Carbonsäuren - oder C4-C18-Dicarbonsäuren

### b-3) wenigstens eine Wachskomponente

Ein Gegenstand der vorliegenden Erfindung sind kosmetische und/oder pharmazeutische Zubereitungen enthaltend
a) wenigstens einen Ester des 2-Propylheptanols mit linearen oder verzweigten, gesättigten oder ungesättigten C4-C36-Carbonsäuren - oder C4-C36-Dicarbonsäuren, vorzugsweise wenigstens einen Ester des 2-Propylheptanols mit linearen oder verzweigten, gesättigten oder ungesättigten C4-C18-Carbonsäuren - oderC4-C18-Dicarbonsäuren

### b-4) wenigstens ein Polymer.

Ein Gegenstand der vorliegenden Erfindung sind kosmetische und/oder pharmazeutische Zubereitungen enthaltend
a) wenigstens einen Ester des 2-Propylheptanols mit linearen oder verzweigten, gesättigten oder ungesättigten C4-C36-Carbonsäuren - oder C4-C36-Dicarbonsäuren, vorzugsweise wenigstens einen Ester des 2-Propylheptanols mit linearen oder verzweigten, gesättigten oder ungesättigten C4-C18-Carbonsäuren - oder C4-C18-Dicarbonsäuren

### b-5) wenigstens einen weiteren Ölkörper

Vorzugsweise enthalten die erfindungsgemäßen Zubereitungen 0,1 bis 80, insbesondere 0,5 bis 70, vorzugsweise 0,75 bis 60 Gew.-%, insbesondere 1 bis 50 Gew.-%, bevorzugt 1 - 40 Gew.% wenigstens eines Esters des 2-Propylheptanols mit linearen oder verzweigten, gesättigten oder ungesättigten C4-C36-Carbonsäuren - oder C4-C36-Dicarbonsäuren.

Ein weiterer Gegenstand der Erfindung sind Zubereitungen kosmetische und/oder pharmazeutische Zubereitungen enthaltend
a) 0,1 - 80 Gew.-%, insbesondere 0,1 bis 70, bevorzugt 0,1 bis 60, insbesondere 0,1 bis 50 Gew.-%, bevorzugt 0,1 - 40 Gew.% wenigstens eines Ester des 2-Propylheptanols mit linearen oder verzweigten, gesättigten oder ungesättigten C4-C36-Carbonsäuren - oder C4-C36-Dicarbonsäuren, vorzugsweise wenigstens eines Esters des 2-Propylheptanols mit linearen oder verzweigten, gesättigten oder ungesättigten C4-C18-Carbonsäuren - oder C4-C18-Dicarbonsäuren
b) 0,1 - 20 Gew.-% Emulgator (b-1) und/oder Tensid (b-2) und/oder Wachskomponente (b-3) und/oder Polymer (b-4) b-5) 0,1 - 40 Gew.-% weiterer Ölkörper und
c) 0 - 98 Gew.-% Wasser.

Die erfindungsgemäßen Zubereitungen enthalten mindestens 0,1, insbesondere mindestens 0,5, insbesondere mindestens 0,75, bevorzugt mindestens 1, bevorzugt mindestens 5 Gew.-% eines oder mehrerer Ester (a).

Alle Gew.-% Angaben beziehen sich auf Gew.-% bezogen auf die kosmetische und/oder pharmazeutische Zubereitung.

In einer bevorzugten Ausführungsform der Erfindung enthalten die Zubereitungen Ester, deren Gesamt C Zahl kleiner gleich 24, bevorzugt kleiner gleich 22 beträgt.

Die erfindungsgemäßen Zubereitungen enthalten bevorzugt Ester von 2-Propylheptanol mit Carbonsäuren ausgewählt aus linearen, verzweigten, gesättigten oder ungesättigten C4 bis C36 Carbonsäuren mit Ausnahme von 2-Propylheptylmethacrylat.

Die erfindungsgemäßen Zubereitungen enthalten bevorzugt Ester des 2-Propylheptanols mit Carbonsäuren, die ausgewählt sind aus den C4 bis C30, insbesondere C6 bis C24, insbesondere C6 bis C22, insbesondere C6 bis C18, insbesondere C8 bis C18 bevorzugt C8 bis C16, vorzugsweise C8 bis C12, insbesondere C6 bis C10 Carbonsäuren oder den entsprechenden Dicarbonsäuren.

Erfindungsgemäß eignen sich für die erfindungsgemäßen Zubereitungen Ester des 2-Propylheptanols mit C4 bis C36, C5 bis C30, C6 bis C26, C7 bis C24, C8 bis C22, C9 bis C20, C10 bis C18, C 11 bis C17, C11 bis C16, C12 bis C15, C13 bis C14 Carbonsäuren oder den entsprechenden Dicarbonsäuren.

In einer besonders bevorzugten Ausführungsform der Erfindung enthalten die erfindungsgemäßen Zubereitungen Ester des 2-Propylheptanols mit Carbonsäuren, die ausgewählt sind aus den C6 bis C12 Carbonsäure sowie Estern des 2-Propylheptanols mit Dicarbonsäuren, die ausgewählt sind aus den C6 bis C12 Dicarbonsäuren, eingesetzt.

Im Sinne der Erfindung sind Zubereitungen bevorzugt, die Ester des 2-Propylheptanols mit C6-C16-, vorzugsweise aus den C6-C12-Carbonsäuren oder den entsprechenden Dicarbonsäuren enthalten. Unter diesen sind lineare, unverzweigte Carbonsäuren bevorzugt. Besonders bevorzugt sind kosmetische Zusammensetzungen mit 2-Propylheptylcaprylat, 2-Propylheptylcapronat und/oder 2-Propylheptylcaprinat.

Die erfindungsgemäßen Zubereitungen enthalten bevorzugt Ester des 2-Propylheptanols mit gesättigten Carbonsäuren.

Die erfindungsgemäßen Zubereitungen enthalten bevorzugt Ester des 2-Propylheptanols mit gesättigten Dicarbonsäuren.

Die Verwendung von Estern des 2-Propylheptanols mit linearen, unverzweigten Carbonsäuren ist erfindungsgemäß bevorzugt.

Die Verwendung von Estern des 2-Propylheptanols mit linearen, unverzweigten Dicarbonsäuren ist erfindungsgemäß bevorzugt.

Die erfindungsgemäßen Zubereitungen können sowohl einzelne Ester aus auch Mischungen verschiedener Ester enthalten.

In einer bevorzugten Ausführungsform der Erfindung enthalten die Zubereitungen mindestens einen Ester gemäß der Anspruch 3.

In einer Ausführungsform der Erfindung enthalten die Zubereitungen mindestens einen Ester ausgewählt aus der Gruppe bestehend aus 2-Propylheptyl-n-hexansäureester, 2-Propylheptyl-n-heptansäureester, 2-Propylheptyl-n-octansäureester,2-Propylheptyl-n-nonansäureester,2-Propylheptyl-n-decansäureester,2-Propylheptyl-n-undecansäureester, 2-Propylheptyl-n-dodecansäureester oder Gemische daraus.

In einer bevorzugten Ausführungsform der Erfindung enthalten die Zubereitungen mindestens einen Ester ausgewählt aus der Gruppe bestehend aus 2-Propylheptyl-n-hexansäureester, 2-Propylheptyl-n-octansäureester, 2-Propylheptyl-n-decansäureester, 2-Propylheptyl-n-dodecansäureester oder Gemische daraus.

In einer Ausführungsform der Erfindung enthalten die Zubereitungen mindestens einen Ester ausgewählt aus der Gruppe bestehend aus 2-Propylheptyl-n-hexansäureester, 2-Propylheptyl-i-hexansäureester, 2-Propylheptyl-n-heptansäureester, 2-Propylheptyl-i-heptansäureester, 2-Propyl-heptyl-n-octansäureester, 2-Propylheptyl-i-octansäureester, 2-Propylheptyl-n-nonansäureester, 2-Propylheptyl-i-nonansäureester, 2-Propylheptyl-n-decansäureester, 2-Propylheptyl-i-decansäureester, 2-Propylheptyl-n-undecansäureester, 2-Propylheptyl-i-undecansäureester, 2-Propylheptyl-n-dodecansäureester, 2-Propylheptyl-i-dodecansäureester oder Gemische daraus.

In einer bevorzugten Ausführungsform der Erfindung enthalten die Zubereitungen mindestens einen Ester ausgewählt aus der Gruppe bestehend aus 2-Propylheptyl-n-hexansäureester, 2-Propylheptyl-i-hexansäureester, 2-Propylheptyl-n-octansäureester, 2-Propylheptyl-i-octansäureester, 2-Propylheptyl-n-decansäureester, 2-Propylheptyl-i-decansäureester, 2-Propylheptyl-n-dodecansäureester, 2-Propylheptyl-i-dodecansäureester oder Gemische daraus.

Eine weitere bevorzugte Ausführungsform der kosmetischen und/oder pharmazeutischen Zubereitungen enthält (a) 0,1-80, insbesondere 0,1 bis 70, vorzugsweise 0,1 bis 60, bevorzugt 0,1 bis 50 Gew.-% wenigstens eines Esters des 2-Propylheptanols mit linearen oder verzweigten, gesättigten oder ungesättigten C4-C36-Carbonsäuren - oder C4-C36-Dicarbonsäuren, (b) 0,1 - 20 Gew.-% Emulgatoren (b-1) und/oder Tenside (b-2) und/oder Wachskomponenten (b-3) und/oder Polymere (b-4), und 0,1 - 40 Gew.-% weiterer Ölkörper (b-5) und (d) 0 - 98 Gew.-% Wasser.

In einer bevorzugten Ausführungsform der Erfindung werden als Ester von 2-Propylheptanol mit C4 bis C36 Dicarbonsäuren Diester verwendet.

In einer bevorzugten Ausführungsform enthalten die erfindungsgemäßen Zubereitungen Ester von 2-Propylheptanol mit linearen, verzweigten, gesättigten oder ungesättigten C4 bis C32 Dicarbonsäuren, insbesondere C4 bis C30, insbesondere C6 bis C24, insbesondere C6 bis C22, insbesondere C8 bis C18, insbesondere C8 bis C16 bevorzugt C8 bis C16, vorzugsweise C8 bis C12 Dicarbonsäuren.

Erfindungsgemäß eignen sich für die erfindungsgemäßen Zubereitungen Ester des 2-Propylheptanols mit C4 bis C36, C5 bis C30, C6 bis C26, C7 bis C24, C8 bis C22, C9 bis C20, C10 bis C18, C 11 bis C17, C11 bis C16, C12 bis C15, C13 bis C14 Dicarbonsäuren.

In einer besonders bevorzugten Ausführungsform der Erfindung enthalten die Zubereitungen Estern des 2-Propylheptanols mit Dicarbonsäuren, die ausgewählt sind aus den C6 bis C12 Dicarbonsäuren.

Estern des 2-Propylheptanols mit gesättigten Dicarbonsäuren sind erfindungsgemäß bevorzugt.

Estern des 2-Propylheptanols mit linearen, unverzweigten Dicarbonsäuren sind erfindungsgemäß bevorzugt.

Als Diester der Dicarbonsäuren von 2-Propylheptanol sind geeignet Di-2-Propylheptyl-n-butandisäurediester, Di-2-Propylheptyl-i-butandisäurediester, Di-2-Propylheptyl-n-pentandisäurediester, Di-2-Propylheptyl-i-pentandisäurediester, Di-2-Propylheptyl-n-hexandisäurediester, Di-2-Propylheptyl-i-hexandisäurediester, Di-2-Propylheptyl-n-heptandisäurediester, Di-2-Propylheptyl-i-heptandisäurediester, Di-2-Propylheptyl-n-octandisäurediester, Di-2-Propylheptyl-i-octandlsäurediester, Di-2-Propylheptyl-n-nonandisäurediester, Di-2- Propylheptyl-i-nonandisäurediester, Di-2-Propylheptyl-n-decandisäurediester, Di-2-Propylheptyl-i-decandisäurediester, Di-2-Propylheptyl-n-undecandisäurediester, Di-2-Propylheptyl-i-undecandisäurediester, Di-2-Propylheptyl-n-undecendisäurediester, Di-2-Propylheptyl-i-undecendisäurediester, Di-2-Propylheptyl-n-dodecandisäurediester, Di-2-Propylheptyl-i-dodecandisäurediester.

Die Diester des Anspruchs 4 sind die erfindungsgemäßen Diester.

Ein weiterer Gegenstand der Erfindung betrifft die Verwendung mindestens eines Esters ausgewählt aus Di-2-Propylheptyl-n-butandisäurediester, Di-2-Propylheptyl-i-butandisäurediester, Di-2-Propylheptyl-n-pentandisäurediester, Di-2-Propylheptyl-i-pentandisäurediester, Di-2-Propylheptyl-n-hexandisäurediester, Di-2-Propyiheptyt-i-hexandisäurediester, Di-2-Propylheptyl-n-heptandisäurediester, Di-2-Propylheptyl-i-heptandisäurediester, Di-2-Propylheptyl-n-octandisäurediester, Di-2-Propylheptyl-i-octandisäurediester, Di-2- Propylheptyl-n-nonandisäurediester, Di-2- Propylheptyl-i-nonandlsäurediester, Di-2-Propylheptyl-n-decandisäurediester, Di-2-Propylheptyl-i-decandisäurediester, Di-2-Propylheptyl-n-undecandisäurediester, Di-2-Propylheptyi-i-undecandi-säurediester, Di-2-Propylheptyl-n-undecendisäurediester, Di-2-Propylheptyl-i-undecendisäurediester, Di-2-Propylheptyl-n-dodecandisäurediester, Di-2-Propylheptyl-i-dodecandisäurediester, 2-Propylheptyl-n-butandisäuremonoester, 2-Propylheptyl-i-butandlsäuremonoester, 2-Propylheptyl-n-pentandisäure-monoester, 2-Propylheptyl-i-pentandisäuremonoester, 2-Propylheptyl-n-hexandisäuremonoester, 2-Propylheptyl-i-hexandisäuremonoester, 2-Propylheptyl-n-heptandisäuremonoester, 2-Propylheptyl-i-heptandisäuremonoester, 2-Propylheptyl-n-octandisäuremonoester, 2-Propylheptyl-i-octandisäuremonoester, 2-Propylheptyl-n-nonandisäuremonoester, 2-Propylheptyl-i-nonandisäuremonoester, 2-Propylheptyl-n-decandisäuremonoester, 2-Propylheptyl-i-decandisäuremonoester, 2-Propylheptyl-n-undecand isäuremonoester, 2-Propylheptyl-i-undecand-isäuremonoester, 2-Propylheptyl-n-undecendisäuremonoester, 2-Propylheptyl-i-undecendisäuremonoester, 2-Propylheptyl-n-dodecandisäuremonoester, 2-Propylheptyl-i-dodecandisäuremonoester oder Mischungen daraus in kosmetischen und/oder pharmazeutischen Zubereitungen.

Ein Gegenstand der Erfindung betrifft Zusammensetzungen, enthaltend Ester von 2-Propylheptanol mit C4 bis C36 Dicarbonsäuren und mindestens einen weiteren Ester ausgewählt aus der Gruppe bestehend aus Estern der C4 bis C 36 Dicarbonsäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen.

Besonders bevorzugt sind Zusammensetzungen, enthaltend Ester von 2-Propylheptanol mit C4 bis C18 Dicarbonsäuren, insbesondere C6 bis C12 Dicarbonsäuren und mindestens einen weiteren Ester ausgewählt aus der Gruppe bestehend aus Estern der C4 bis C 18, insbesondere C6 bis C12 Dicarbonsäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen.

Ein weiterer Gegenstand der Erfindung betrifft Zusammensetzungen, enthaltend Ester von 2-Propylheptanol von n-Butandisäure und mindestens einen weiteren Ester ausgewählt aus der Gruppe bestehend aus Estern von n-Butandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen. In einer bevorzugten Ausführungsform der Erfindung enthält die Zusammensetzung jeweils die Diester der Disäuren, so z.B. Di-2-Propylheptyl-n-butandisaurediester und Di-5-Methyl-2-propylhexyl-n-butandisäurediester.

Ein weiterer Gegenstand der Erfindung betrifft Zusammensetzungen, enthaltend Ester von 2-Propylheptanol von i-Butandisäure und mindestens einen weiteren Ester ausgewählt aus der Gruppe bestehend aus Estern von i-Butandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von n-Pentandisäure und mindestens einen weiteren Ester ausgewählt aus der Gruppe bestehend aus Estern von n-Pentandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von i-Pentandisäure und mindestens einen weiteren Ester ausgewählt aus der Gruppe bestehend aus Estern von i-Pentandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von n-Hexandisäure und mindestens einen weiteren Ester ausgewählt aus der Gruppe bestehend aus Estern von n-Hexandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von i-Hexandisäure und mindestens einen weiteren Ester ausgewählt aus der Gruppe bestehend aus Estern von i-Hexandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus, sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von n-Heptandisäure und mindestens einen weiteren Ester ausgewählt aus der Gruppe bestehend aus Estern von n-Heptandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus, sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von i-Heptandisäure und mindestens einen weiteren Ester ausgewählt aus der Gruppe bestehend aus Estern von i-Heptandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus, sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von n-Octandisäure und mindestens einen weiteren Ester ausgewählt aus der Gruppe bestehend aus Estern von n-Octandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus, sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von i-Octandisäure und mindestens einen weiteren Ester ausgewählt aus der Gruppe bestehend aus Estern von i-Octandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus, sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von n-Nonandisäure und mindestens einen weiteren Ester ausgewählt aus der Gruppe bestehend aus Estern von n-Nonandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus, sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von i-Nonandisäure und mindestens einen weiteren Ester ausgewählt aus der Gruppe bestehend aus Estern von i-Nonandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus, sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von n-Decandisäure und mindestens einen weiteren Ester ausgewählt aus der Gruppe bestehend aus Estern von n-Decandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus, sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von i-Decandisäure und mindestens einen weiteren Ester ausgewählt aus der Gruppe bestehend aus Estern von i-Decandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von n-Undecandisäure und mindestens einen weiteren Ester ausgewählt aus der Gruppe bestehend aus Estern von n-Undecandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von i-Undecandisäure und mindestens einen weiteren Ester ausgewählt aus der Gruppe bestehend aus Estern von i-Undecandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von Undecendisäure und mindestens einen weiteren Ester ausgewählt aus der Gruppe bestehend aus Estern von Undecendisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von n-Dodecandisäure und mindestens einen weiteren Ester ausgewählt aus der Gruppe bestehend aus Estern von n-Dodecandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von i-Dodecandisäure und mindestens einen weiteren Ester ausgewählt aus der Gruppe bestehend aus Estern von i-Dodecandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von n-Tridecandisäure und mindestens einen weiteren Ester ausgewählt aus der Gruppe bestehend aus Estern von n-Tridecandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von i-Tridecandisäure und mindestens einen weiteren Ester ausgewählt aus der Gruppe bestehend aus Estern von i-Tridecandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von n-Tetradecandisäure und mindestens einen weiteren Ester ausgewählt aus der Gruppe bestehend aus Estern von n-Tetradecandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von i-Tretradecandisäure und mindestens einen weiteren Ester ausgewählt aus der Gruppe bestehend aus Estern von i-Tetradecandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von n-Pentadecandisäure und mindestens einen weiteren Ester ausgewählt aus der Gruppe bestehend aus Estern von n-Pentadecandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von i-Pentadecandisäure und mindestens einen weiteren Ester ausgewählt aus der Gruppe bestehend aus Estern von i-Pentadecandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von n-Hexadecandisäure und mindestens einen weiteren Ester ausgewählt aus der Gruppe bestehend aus Estern von n-Hexadecandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von i-Hexadecandisäure und mindestens einen weiteren Ester ausgewählt aus der Gruppe bestehend aus Estern von i-Hexadecandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von n-Heptadecandisäure und mindestens einen weiteren Ester ausgewählt aus der Gruppe bestehend aus Estern von n-Heptadecandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von i-Heptadecandisäure und mindestens einen weiteren Ester ausgewählt aus der Gruppe bestehend aus Estern von i-Heptadecandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von n-Octadecandisäure und mindestens einen weiteren Ester ausgewählt aus der Gruppe bestehend aus Estern von n-Octadecandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von i-Octadecandisäure und mindestens einen weiteren Ester ausgewählt aus der Gruppe bestehend aus Estern von i-Octadecandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von n-Nonadecandisäure und mindestens einen weiteren Ester ausgewählt aus der Gruppe bestehend aus Estern von n-Nonadecandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von i-Nonadecandisäure und mindestens einen weiteren Ester ausgewählt aus der Gruppe bestehend aus Estern von i-Nonadecandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von n-Eicosandisäure und mindestens einen weiteren Ester ausgewählt aus der Gruppe bestehend aus Estern von n-Eicosandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von i-Eicosandisäure und mindestens einen weiteren Ester ausgewählt aus der Gruppe bestehend aus Estern von i-Eicosandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von n-Docosandisäure und mindestens einen weiteren Ester ausgewählt aus der Gruppe bestehend aus Estern von n-Docosandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von i-Docosandisäure und mindestens einen weiteren Ester ausgewählt aus der Gruppe bestehend aus Estern von i-Docosandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von n-Tetracosandisäure und mindestens einen weiteren Ester ausgewählt aus der Gruppe bestehend aus Estern von n-Tetracosandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von i-Tetracosandisäure und mindestens einen weiteren Ester ausgewählt aus der Gruppe bestehend aus Estern von i-Tetracosandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von n-Hexacosandisäure und mindestens einen weiteren Ester ausgewählt aus der Gruppe bestehend aus Estern von n-Hexacosandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein weiterer Gegenstand der Erfindung betrifft Zusammensetzungen, enthaltend Ester von 2-Propylheptanol von i-Hexacosandisäure und mindestens einen weiteren Ester ausgewählt aus der Gruppe bestehend aus Estern von i-Hexacosandisäure mit mindestens einem Alkohol ausgewählt aus der Gruppe bestehend aus 3-Methyl-2-Propylhexanol, 4-Methyl-2-propylhexanol und 5-Methyl-2-propylhexanol und Mischungen daraus sowie ihre Verwendung in kosmetischen und/oder pharmazeutischen Zubereitungen. In einer bevorzugten Ausführungsform der Erfindung enthält die Zusammensetzung jeweils die Diester der Disäuren.

Ein Gegenstand der Erfindung betrifftEstervon2-Propylheptanol mitDimer-Fettsäuren. Der Begriff "Dimer-Fettsäuren" bezeichnet Polycarbonsäuren, die durch Polymerisation ungesättigter Fettsäuren, vornehmlich der Ölsäure oder der Tallölfettsäure gewonnen werden. Kommerzielle Dimer-Fettsäuren bestehen aus einem Gemisch, das neben geringen Anteilen linearer u. verzweigter C18 Monocarbonsäuren (Monomerfettsäure) überwiegend C36 Dicarbonsäure u. unterschiedlich hohe Anteile C54 Tricarbonsäure (Trimerfettsäure) neben Spuren höherer Polymerfettsäuren enthält.

Ein Gegenstand der Erfindung betrifft Zusammensetzungen, enthaltend Ester von 2-Propylheptanol mit Dimer-Fettsäuren und mindestens einen weiteren Ester ausgewählt aus der Gruppe bestehend aus den Estern von 3-Methyl-2-propylhexanol mit Dimer-Fettsäuren, den Estern von 4-Methyl-2-propylhexanol mit Dimer-Fettsäuren, den Estern von 5-Methyl-2-propylhexanol mit Dimer-Fettsäuren und Mischungen daraus und Mischungen daraus sowie ihre Verwendung in kosmetischen und/oder pharmazeutischen Zubereitungen. In einer bevorzugten Ausführungsform der Erfindung enthält die Zusammensetzung jeweils die Diester der Disäuren.

Die erfindungsgemäßen Zubereitungen, die erfindungsgemäßen Zusammensetzungen sowie die Ester gemäß der Erfindung eignen sich als Basis in allen kosmetischen Mittel zur Körperpflege und -reinigung wie z.B. Körperöl, Babyöl, Körpermilch, Cremes, Lotionen, sprühbare Emulsionen, Sonnenschutzmittel, Antitranspirantien, Flüssig- und Stückseifen etc. eingearbeitet werden. Sie lassen sich auch in tensidhaltigen Formulierungen wie z.B. Schaum- und Duschbädern, Haarshampoos und Pflegespülungen einsetzen. Sie lassen sich als Pflegekomponente auf Tissues, Papiere, Wipes, Vlies-Produkte, Schwämme, Puffs, Pflaster und Bandagen applizieren, die ihren Einsatz im Bereich der Hygiene und Pflegefinden (Feuchttücher zur Baby-Hygiene und Baby-Pflege, Reinigungstücher, Gesichtreinigungstücher, Hautpflegetücher, Pflegetücher mit Wirkstoffen gegen die Hautalterung, Wipes mit Sonnenschutzformulierungen und Insektenrepellentien sowie Wipes zur dekorative Kosmetik oder zum After-Sun-Treatment, Toiletten-Feuchttücher, Antitranspirant-Wipes, Windeln, Taschentücher, Wet Wipes, Hygieneprodukte, Selbstbräunungs Wipes). Sie lassen sich u.a. auch in Zubereitungen zur Haarpflege, Haarreinigung oder Haarfärbung einsetzen.

Je nach Applikationszweck enthalten die kosmetischen Formulierungen eine Reihe weiterer Hilfs- und Zusatzstoffe, wie beispielsweise Tenside, weitere Ölkörper, Emulgatoren, Perlglanzwachse, Konsistenzgeber, Verdickungsmittel, Überfettungsmittel, Stabilisatoren, Polymere, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosinaseinhibitoren (Depigmentierungsmittel), Hydrotrope, Solubilisatoren, Konservierungsmittel, Parfümöle, Farbstoffe etc., die nachstehend exemplarisch aufgelistet sind.

### Emulgator b-1)

In einer Ausführungsform der Erfindung enthalten die erfindungsgemäßen Zubereitungen mindestens einen Emulgator. Die erfindungsgemäßen Zusammensetzungen enthalten den/die Emulgator(en) in einer Menge von 0 bis 40 Gew.-%, vorzugsweise 0,1 bis 20 Gew.-% vorzugsweise 0,1 bis 15 Gew.-% und insbesondere 0,1 bis 10 Gew.-% bezogen auf das Gesamtgewicht der Zusammensetzung.

In einer Ausführungsform der Erfindung enthält die erfindungsgemäße Zubereitung mehr als einen Emulgator. Der Fachmann setzt in Abhängigkeit der übrigen Komponenten übliche Emulgator Systeme (wie z.B. Emulgator und Co-Emulgator) ein.

### Nicht-ionische Emulgatoren

Zur Gruppe der nicht-ionischen Emulgatoren gehören beispielsweise:
(1) Anlagerungsprodukte von 2 bis 50 Mol Ethylenoxid und/oder 1 bis 20 Mol Propylenoxid an lineare Fettalkohole mit 8 bis 40 C-Atomen, an Fettsäuren mit 12 bis 40 C-Atomen und an Alkylphenole mit 8 bis 15 C-Atomen in der Alkylgruppe.
(2) C₁₂-C₁₈-Fettsäuremono- und -diester von Anlagerungsprodukten von 1 bis 50 Mol Ethylenoxid an Glycerin.
(3) Sorbitanmono- und -diester von gesättigten und ungesättigten Fettsäuren mit 6 bis 22 Kohlenstoffatomen und deren Ethylenoxidanlagerungsprodukte.
(4) Alkylmono- und -oligoglycoside mit 8 bis 22 Kohlenstoffatomen im Alkylrest und deren ethoxylierte Analoga.
(5) Anlagerungsprodukte von 7 bis 60 Mol Ethylenoxid an Ricinusöl und/oder gehärtetes Ricinusöl.
(6) Polyol- und insbesondere Polyglycerinester, wie z. B. Polyolpoly-12-hydroxystearate, Polyglycerinpolyricinoleat, Polyglycerindiisostearat oder Polyglycerindimerat. Ebenfalls geeignet sind Gemische von Verbindungen aus mehreren dieser Substanzklassen.
(7) Anlagerungsprodukte von 2 bis 15 Mol Ethylenoxid an Ricinusöl und/oder gehärtetes Ricinusöl.
(8) Partialester auf Basis linearer, verzweigter, ungesättigter bzw. gesättigter C₆-C₂₂- Fettsäuren, Ricinolsäure sowie 12-Hydroxystearinsäure und Polyglycerin, Pentaerythrit, Dipentaerythrit, Zuckeralkohole (z. B. Sorbit), Alkylglucoside (z. B. Methylglucosid, Butylglucosid, Laurylglucosid) sowie Polyglucoside (z. B. Cellulose), oder Mischester wie z. B. Glycerylstearatcitrat und Glycerylstearatlactat.
(9) Polysiloxan-Polyalkyl-Polyether-Copolymere bzw. entsprechende Derivate.
(10) Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. Je nach Ethoxylierungsgrad handelt es sich um W/O- oder O/W-Emulgatoren. C_{12/18}-Fettsäuremono- und -diester von Anlagerungsprodukten von Ethylenoxid an Glycerin sind als Rückfettungsmittel für kosmetische Zubereitungen bekannt.

Erfindungsgemäß besonders gut geeignete und milde Emulgatoren sind Polyolpoly-12- hydroxystearate und Abmischungen davon, welche beispielsweise unter den Marken "Dehymuls® PGPH" (W/O-Emulgator) oder "Eumulgin® VL 75" (Abmischung mit Coco Glucosides im Gewichtsverhältnis 1:1, O/W-Emulgator) oder Dehymuls® SBL (W/O-Emulgator) von der Cognis Deutschland GmbH vertrieben werden. In diesem Zusammenhang sei insbesondere auf das Europäische Patent EP 0 766 661 B1 verwiesen. Die Polyolkomponente dieser Emulgatoren kann sich von Stoffen ableiten, die über mindestens zwei, vorzugsweise 3 bis 12 und insbesondere 3 bis 8 Hydroxylgruppen und 2 bis 12 Kohlenstoffatome verfügen.

Als lipophile W/O-Emulaatoren eignen sich prinzipiell Emulgatoren mit einem HLB-Wert von 1 bis 8, die in zahlreichen Tabellenwerken zusammengefaßt und dem Fachmann bekannt sind. Einige dieser Emulgatoren sind beispielsweise in Kirk-Othmer, "Encyclopedia of Chemical Technology", 3. Aufl., 1979, Band 8, Seite 913, aufgelistet. Für ethoxylierte Produkte lässt sich der HLB-Wert auch nach folgender Formel berechnen: HLB = (100 - L) : 5, wobei L der Gewichtsanteil der lipophilen Gruppen, d. h. der Fettalkyl- oder Fettacylgruppen in Gewichtsprozent, in den Ethylenoxidaddukten ist.

Besonders vorteilhaft aus der Gruppe der W/O-Emulgatoren sind Partialester von Polyolen, insbesondere von C₄-C₆-Polyolen, wie beispielsweise Partialester des Pentaerythrits oder Zuckerestem, z. B. Saccharosedistearat, Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Als Emulgatoren geeignet sind auch Anlagerungsprodukte von 1 bis 30, vorzugsweise 5 bis 10 Mol Ethylenoxid an die genannten Sorbitanester.

Je nach Formulierung kann es vorteilhaft sein, zusätzlich wenigstens einen Emulgator aus der Gruppe nicht-ionischer O/W-Emulgatoren (HLB-Wert: 8 - 18) und/oder Solubilisatoren einzusetzen. Hierbei handelt es sich beispielsweise um die bereits einleitend erwähnten Ethylenoxid-Addukte mit einem entsprechend hohen Ethoxylierungsgrad, z. B. 10-20 Ethylenoxid-Einheiten für O/W-Emulgatoren und 20-40 Ethylenoxid-Einheiten für sogenannte Solubilisatoren. Erfindungsgemäß besonders vorteilhaft als O/W-Emulgatoren sind Ceteareth-12 und PEG-20 Stearat. Als Solubilisatoren bevorzugt geeignet sind Eumulgin® HRE 40 (INCI: PEG-40 Hydrogenated Castor Oil), Eumulgin® HRE 60 (INCI: PEG-60 Hydrogenated Castor Oil), Eumulgin® L (INCI: PPG-1-PEG-9 Laurylglycolether), sowie Eumulgin® SML 20 (INCI: Polysorbat-20).

Nicht-ionische Emulgatoren aus der Gruppe der Alkyloligoglycoside sind besonders hautfreundlich und daher bevorzugt als O/W-Emulgatoren geeignet. C₈-C₂₂-Alkylmono- und-oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 22 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt. Produkte, die unterder Bezeichnung Plantacare® zur Verfügung stehen, enthalten eine glucosidisch gebundene C₈-C₁₆-Alkylgruppe an einem Oligoglucosidrest, dessen mittlerer Oligomerisationsgrad bei 1 bis 2 liegt. Auch die vom Glucamin abgeleiteten Acylglucamide sind als nicht-ionische Emulgatoren geeignet. Erfindungsgemäß bevorzugt ist ein Produkt, das unter der Bezeichnung Emulgade® PL 68/50 von der Cognis Deutschland GmbH vertrieben und ein 1:1-Gemisch aus Alkylpolyglucosiden und Fettalkoholen darstellt. Erfindungsgemäß vorteilhaft einsetzbar ist auch ein Gemisch aus Lauryl Glucoside, Polyglyceryl-2-Dipolyhydroxystearate, Glycerin und Wasser, das unter der Bezeichnung Eumulgin® VL 75 im Handel ist.

Als Emulgatoren kommen weiterhin Substanzen, wie Lecithine und Phospholipide in Frage. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Tenside b-2)

In einer Ausführungsform der Erfindung enthalten die erfindungsgemäßen Zubereitungen mindestens ein Tensid. Als oberflächenaktive Stoffe können anionische, nichtionische, kationische und/oder amphotere bzw. zwitterionische Tenside enthalten sein. In tensidhaltigen kosmetischen Zubereitungen, wie beispielsweise Duschgelen, Schaumbädern, Shampoos etc. ist vorzugsweise wenigstens ein anionisches Tensid enthalten.

Die erfindungsgemäßen Zusammensetzungen enthalten den/die Tenside in einer Menge von 0 bis 40 Gew.-%., vorzugsweise 0 bis 20 Gew.-%, vorzugsweise 0,1 bis 15 Gew.-% und insbesondere 0,1 bis 10 Gew.-% bezogen auf das Gesamtgewicht der Zusammensetzung.

Typische Beispiele für **nichtionische Tenside** sind Fettalkoholpolyglycolether, Alkylphenolpolyglycolether, Fettsäurepolyglycolester, Fettsäureamidpolyglycolether, Fettamin-polyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen.

Als **zwitterionische Tenside** werden solche oberflächenaktiven Verbindungen bezeichnet, die im Molekül mindestens eine quartäre Ammoniumgruppe und mindestens eine -COOH- oder -SO₃⁽⁻⁾-Gruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxylmethyl-3-hydroxyethylimidazolin mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls, insbesondere alsCo-Tensidegeeignet, sind ampholytischeTenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

**Anionische Tenside** sind gekennzeichnet durch eine wasserlöslich machende, anionische Gruppe wie z. B. eine Carboxylat-, Sulfat-, Sulfonat- oder Phosphat-Gruppe und einen lipophilen Rest. Hautverträgliche anionische Tenside sind dem Fachmann in großer Zahl aus einschlägigen Handbüchern bekannt und im Handel erhältlich. Es handelt sich dabei insbesondere um Alkylsulfate in Form ihrer Alkali-, Ammonium- oder Alkanolammoniumsalze, Alkylethersulfate, Alkylethercarboxylate, Acylisethionate, Acylsarkosinate, Acyltaurine mit linearen Alkyl- oder Acylgruppen mit 12 bis 18 C-Atomen sowie Sulfosuccinate und Acylglutamate in Form ihrer Alkali- oder Ammoniumsalze.

Als **kationische Tenside** sind insbesondere quartäre Ammoniumverbindungen verwendbar. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können die sehr gut biologisch abbaubaren quaternären Esterverbindungen, wie beispielsweise die unter dem Warenzeichen Stepantex® vertriebenen Dialkylammoniummethosulfate und Methylhydroxyalkyldialkoyloxyalkylammoniummethosulfate und die entsprechenden Produkte der Dehyquart®-Reihe, als kationische Tenside eingesetzt werden. Unter der Bezeichnung "Esterquats" werden im allgemeinen quaternierte Fettsäuretriethanolaminestersalze verstanden. Sie können den erfindungsgemäßen Zusammensetzungen einen besonderen Weichgriff verleihen. Es handelt sich dabei um bekannte Stoffe, die man nach den einschlägigen Methoden derorganischen Chemie herstellt. Weitere erfindungsgemäß verwendbare kationische Tensidestellen die quatemisierten Proteinhydrolysate dar.

### Wachskomponente b-3)

In einer Ausführungsform der Erfindung enthalten die erfindungsgemäßen Zubereitungen mindestens eine Wachskomponente. Die erfindungsgemäßen Zusammensetzungen enthalten den/die Wachskomponente(n) in einer Menge von 0 bis 40 Gew.-%, insbesondere von 0 bis 20 Gew.-%, vorzugsweise 0,1 bis 15 Gew.-% und insbesondere 0,1 bis 10 Gew.-% bezogen auf das Gesamtgewicht der Zusammensetzung.

Unter dem Begriff Wachs werden üblicherweise alle natürlichen oder künstlich gewonnenen Stoffe und Stoffgemische mitfolgenden Eigenschaften verstanden: sie sind von fester bis brüchig harter Konsistenz, grob bis feinkristallin, durchscheinend bis trüb und schmelzen oberhalb von 30°C ohne Zersetzung. Sie sind schon wenig oberhalb des Schmelzpunktes niedrigviskos und nicht fadenziehend und zeigen eine stark temperaturabhängige Konsistenz und Löslichkeit. Erfindungsgemäß einsetzbar ist eine Wachskomponente oder ein Gemisch von Wachskomponenten, die bei 30 C oder darüber schmelzen.

Als Wachse können erfindungsgemäß auch Fette und fettähnliche Substanzen mit wachsartiger Konsistenz eingesetzt werden, solange sie den geforderten Schmelzpunkt haben. Hierzu gehören u.a. Fette (Triglyceride), Mono- und Diglyceride, natürliche und synthetische Wachse, Fett- und Wachsalkohole, Fettsäuren, Ester von Fettalkoholen und Fettsäuren sowie Fettsäureamide oder beliebige Gemische dieser Substanzen.

Unter Fetten versteht man Triacylglycerine, also die Dreifachester von Fettsäuren mit Glycerin. Bevorzugt enthalten sie gesättigte, unverzweigte und unsubstituierte Fettsäurereste. Hierbei kann es sich auch um Mischester, also um Dreifachester aus Glycerin mit verschiedenen Fettsäuren handeln. Erfindungsgemäß einsetzbar und als Konsistenzgeber besonders gut geeignet sind so genannte gehärtete Fette und Öle, die durch Partialhydrierung gewonnen werden. Pflanzliche gehärtete Fette und Öle sind bevorzugt, z. B. gehärtetes Rizinusöl, Erdnußöl, Sojaöl, Rapsöl, Rübsamenöl, Baumwollsaatöl, Sojaöl, Sonnenblumenöl, Palmöl, Palmkernöl, Leinöl, Mandelöl, Maisöl, Olivenöl, Sesamöl, Kakaobutter und Kokosfett.

Geeignet sind u.a. die Dreifachester von Glycerin mit C12-C60-Fettsäuren und insbesondere C12-C36-Fettsäuren. Hierzu zählt gehärtetes Rizinusöl, ein Dreifachester aus Glycerin und einer Hydroxystearinsäure, der beispielsweise unter der Bezeichnung Cutina HR im Handel ist. Ebenso geeignet sind Glycerintristearat, Glycerintribehenat (z. B. Syncrowax HRC), Glycerintripalmitat oder die unter der Bezeichnung Syncrowax HGLC bekannten Triglycerid-Gemische, mit der Vorgabe, dass der Schmelzpunkt der Wachskomponente bzw. des Gemisches bei 30 °C oder darüber liegt.

Als Wachskomponenten sind erfindungsgemäß insbesondere Mono- und Diglyceride bzw. Mischungen dieser Partialglyceride einsetzbar. Zu den erfindungsgemäß einsetzbaren Glyceridgemischen zählen die von der Cognis Deutschland GmbH & Co. KG vermarkteten Produkte Novata AB und Novata B (Gemisch aus C12-C18-Mono-, Di- und Triglyceriden) sowie Cutina MD oder Cutina GMS (Glycerylstearat).

Zu den erfindungsgemäß als Wachskomponente einsetzbaren Fettalkoholen zählen die C12-C50-Fettalkohole. Die Fettalkohole können aus natürlichen Fetten, Ölen und Wachsen gewonnen werden, wie beispielsweise Myristylalkohol, 1-Pentadecanol, Cetylalkohol, 1-Heptadecanol, Stearylalkohol, 1-Nonadecanol, Arachidylalkohol, 1-Heneicosanol, Behenylalkohol, Brassidylalkohol, Lignocerylalkohol, Cerylalkohol oder Myricylalkohol. Erfindungsgemäß bevorzugt sind gesättigte unverzweigte Fettalkohole. Aber auch ungesättigte, verzweigte oder unverzweigte Fettalkohole können erfindungsgemäß als Wachskomponente verwendet werden, solange sie den geforderten Schmelzpunkt aufweisen. Erfindungsgemäß einsetzbar sind auch Fettalkoholschnitte, wie sie bei der Reduktion natürlich vorkommender Fette und Öle wie z. B. Rindertalg, Erdnußöl, Rüböl, Baumwollsaatöl, Sojaöl, Sonnenblumenöl, Palmkernöl, Leinöl, Rizinusöl, Maisöl, Rapsöl, Sesamöl, Kakaobutter und Kokosfett anfallen. Es können aber auch synthetische Alkohole, z. B. die linearen, geradzahligen Fettalkohole der Ziegler-Synthese (Alfole) oder die teilweise verzweigten Alkohole aus der Oxosynthese (Dobanole) verwendet werden. Erfindungsgemäß besonders bevorzugt geeignet sind C14-C22-Fettalkohole, die beispielsweise von der Cognis Deutschland GmbH unter der Bezeichnung Lanette 16 (C16-Alkohol), Lanette 14 (C14-Alkohol), Lanette O (C16/C18-Alkohol) und Lanette 22 (C18/C22-Alkohol) vermarktet werden. Fettalkohole verleihen den Zusammensetzungen ein trockeneres Hautgefühl als Triglyceride und sind daher gegenüber letzteren bevorzugt.

Als Wachskomponenten können auch C14-C40-Fettsäuren oder deren Gemische eingesetzt werden. Hierzu gehören beispielsweise Myristin-, Pentadecan-, Palmitin-, Margarin-, Stearin-, Nonadecan-, Arachin-, Behen-, Lignocerin-, Cerotin-, Melissin-, Eruca- und Elaeostearinsäure sowie substituierte Fettsäuren, wie z. B. 12-Hydroxystearinsäure, und die Amide oder Monoethanolamide der Fettsäuren, wobei diese Aufzählung beispielhaften und keinen beschränkenden Charakter hat.

Erfindungsgemäß verwendbar sind beispielsweise natürliche pflanzliche Wachse, wie Candelillawachs, Carnaubawachs, Japanwachs, Espartograswachs, Korkwachs, Guarumawachs, Reiskeimölwachs, Zuckerrohrwachs, Ouricurywachs, Montanwachs, Sonnenblumenwachs, Fruchtwachse wie Orangenwachse, Zitronenwachse, Grapefruitwachs, Lorbeerwachs (=Bayberrywax) und tierische Wachse, wie z. B. Bienenwachs, Schellackwachs, Walrat, Wollwachs und Bürzelfett. Im Sinne der Erfindung kann es vorteilhaft sein, hydrierte oder gehärtete Wachse einzusetzen. Zu den erfindungsgemäß verwendbaren natürlichen Wachsen zählen auch die Mineralwachse, wie z. B. Ceresin und Ozokerit oder die petrochemischen Wachse, wie z. B. Petrolatum, Paraffinwachse und Mikrowachse. Als Wachskomponente sind auch chemisch modifizierte Wachse, insbesondere die Hartwachse, wie z. B. Montanesterwachse, Sasolwachse und hydrierte Jojobawachse einsetzbar. Zu den synthetischen Wachsen, die erfindungsgemäß einsetzbar sind, zählen beispielsweise wachsartige Polyalkylenwachse und Polyethylenglycolwachse. Pflanzliche Wachse sind erfindungsgemäß bevorzugt.

Die Wachskomponente kann ebenso gewählt werden aus der Gruppe derWachsester aus gesättigten und/oder ungesättigten, verzweigten und/oder unverzweigten Alkancarbonsäuren und gesättigten und/oder ungesättigten, verzweigten und/oder unverzweigten Alkoholen, aus der Gruppe der Ester aus aromatischen Carbonsäuren, Dicarbonsäuren, Tricarbonsäuren bzw. Hydroxycarbonsäuren (z. B. 12-Hydroxystearinsäure) und gesättigten und/oder ungesättigten, verzweigten und/oder unverzweigten Alkoholen, sowie ferner aus der Gruppe der Lactide langkettiger Hydroxycarbonsäuren. Beispiel solcher Ester sind die C16-C40-Alkylstearate, C20-C40-Alkylstearate (z. B. Kesterwachs K82H), C20-C40-Dialkylester von Dimersäuren, C18-C38-Alkylhydroxystearoylstearate oder C20-C40- Alkylerucate. Ferner sind C30-C50-Alkylbienenwachs, Tristearylcitrat, Triisostearylcitrat, Stearylheptanoat, Stearyloctanoat, Trilaurylcitrat, Ethylenglycoldipalmitat, Ethylenglycoldistearat, Ethylenglykoldi(12-hydroxystearat), Stearylstearat, Palmitylstearat, Stearylbehenat, Cetylester, Cetearylbehenat und Behenylbehenat einsetzbar.

### Polymere b-4)

In einer Ausführungsform der Erfindung enthalten die erfindungsgemäßen Zubereitungen mindestens ein Polymer. Die erfindungsgemäßen Zusammensetzungen enthalten das/die Polymere(n) in einer Menge von 0 bis 20 Gew.-%, vorzugsweise 0,1 bis 15 Gew.-% und insbesondere 0,1 bis 10 Gew.-% bezogen auf das Gesamtgewicht der Zusammensetzung.

Geeignete **kationische Polymere** sind beispielsweise kationische Cellulosederivate, wie z. B. eine quatemierte Hydroxyethylcellulose, die unter der Bezeichnung Polymer JR 400® von Amerchol erhältlich ist, kationische Stärke, Copolymerevon Diallylammoniumsalzen undAcrylamiden, quatemierte VinylpyrrolidonNinylimidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed collagen (Lamequat®L/Grünau), quatemierte Weizenpolypeptide, Polyethylenimin, kationische Siliconpolymere, wie z. B. Amidomethicone, Copolymere der Adipinsäure und Dimethylaminohydroxypropyldiethylentriamin (Cartaretine®/Sandoz), Copolymereder Acrylsäure mit Dimethyldiallylammoniumchlorid (Merquat® 550/Chemviron), Polyaminopolyamide, kationische Chitinderivate wie beispielsweise quatemiertes Chitosan, gegebenenfalls mikrokristallin verteilt, Kondensationsprodukte aus Dihalogenalkylen, wie z.B. Dibrombutan mit Bisdialkylaminen, wie z. B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z. B. Mirapol® A-15, Mirapol® AD-1, Mirapol® AZ-1 der Firma Miranol.

Als **anionische, zwitterionische, amphotere und nichtionische Polymere** kommen beispielsweise Vinylaceta/Crotonsäure-Copolymere, VinylpyrrolidonNinylacrylat-Copolymere, Vinylacetat/Butylmaleat/Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/Acrylat-Copolymere, Octylacrylamid/Methylmethacrylat/ tert.Butylaminoethylmethacrylat/2-Hydroxypropylmethacrylat-Copolymere, Polyvinylpyrrolidon, Vinylpyrrolidon/Vinylacetat-Copolymere, VinylpyrrolidonlDimethylaminoethylmethacrylat/Vinylcaproactam-Terpolymere sowie gegebenenfalls derivatisierte Celluloseether und Silicone in Frage.

Als Polymere eigen sich ebenso Polysaccharide, insbesondere Xanthan-Gum, Guar-Guar, Agar-Agar, Alginate und Tylosen.

### Weitere Ölkörper b-5)

Körperpflegemittel, wie Cremes, Körperöle, Lotionen und Milchen, enthalten üblicherweise eine Reihe weiterer Ölkörper und Emollients, die dazu beitragen, die sensorischen Eigenschaften weiter zu optimieren. Die Ölkörper (erfindungsgemäße Ester plus weitere Ölkörper) sind üblicherweise in einer Gesamtmenge von 0,1 - 80, insbesondere 0,5 bis 70, bevorzugt 1 bis 60, insbesondere 1 bis 50 Gew.-%, insbesondere 1 bis 40 Gew.-%, vorzugsweise 5 - 25 Gew.-% und insbesondere 5 -15 Gew.-% enthalten. Die weiteren Ölkörper sind üblicherweise in einer Menge von 0,1 bis 40 Gew.-% enthalten

Als weitere Ölkörper kommen beispielsweise Guerbetalkohole auf Basis von Fettalkoholen mit 6 bis 18, vorzugsweise 8 bis 10 Kohlenstoffatomen, in Frage sowie weitere, zusätzliche Ester wie Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z. B. Propylenglycol, Dimerdiol oder Trimertriol), Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂Fettalkoholcarbonate, wie z. B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkohalen (z. B. Finsolv®TN), lineare oder verzweigte, symmetrischeoderunsymmetrische Dialkylethermit6 bis22 Kohlenstoffatomen pro Alkylgruppe,wie z.B. Dicaprylylether (Cetiol® OE), Ringöffnungsprodukte von epoxidierten Fettsäureestern mit Polyolen und Kohlenwasserstoffen oder deren Gemischen (Cetiol® DD).

### Weitere Inhaltsstoffe

Als **Verdickungsmittel** eignen sich beispielsweise Aerosil-Typen (hydrophile Kieselsäuren), Carboxymethylcellulose und Hydroxyethyl- und Hydroxypropylcellulose, Polyvinylalkohol, Polyvinylpyrrolidon und Bentonite wie z. B. Bentone® Gel VS-5PC (Rheox).

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z. B. Wärme wieder abzugeben. UV-B-Filter können öllöslich oder wasserlöslich sein. Als typische UV-A-Filter kommen insbesondere Derivate des Benzoylmethans in Frage. Die UV-A und UV-B-Filter können selbstverständlich auch in Mischungen eingesetzt werden, z.B. Kombinationen aus den Derivaten des Benzoylmethans, z. B. 4-tert.-Butyl-4'-methoxydibenzoylmethan (Parsol® 1789) und 2-Cyano-3,3-phenylzimtsäure-2-ethyl-hexylester (Octocrylene) sowie Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Häufig werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen auch unlösliche Lichtschutzpigmente, nämlich feindisperse Metalloxide in Frage. Beispiele für geeignete Metalloxide sind insbesondere Zinkoxid und Titandioxid. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt.

Unter **biogenen Wirkstoffen** sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitaminkomplexe zu verstehen.

**Desodorierende Wirkstoffe** wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend eignen sich als deosodorierende Wirkstoffe u.a. keimhemmende Mittel, Enzyminhibitoren, Geruchsabsorber oder Geruchsüberdecker.

Als **Insekten-Repellentien** kommen beispielsweise N,N-Diethyl-m-toluamid, 1 ,2-Pentandioloder 3-(N-n-Butyl-N-acetyl-amino)-propionsäureethylester), welches unter der Bezeichnung Insect Repellent® 3535 von der Merck KGaA vertrieben wird, sowie Butylacetylaminopropionate in Frage.

Als **Selbstbräuner** eignetsich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

Als **Konservierungsmittel** eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

Als ParfümöleseiengenanntGemischeaus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.

Als **Perlglanzwachse,** insbesondere für den Einsatz in tensidischen Formulierungen, kommen beispielsweise in Frage: Alkylenglycdlester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit6bis22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Stabilisatoren** können Metallsalze von Fettsäuren, wie z. B. Magnesium-, Aluminium- und/oder Zinkstearat bzw. -ricinoleat eingesetzt werden.

Zur Verbesserung des Fließverhaltens können ferner **Hydrotrope,** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein.

Die erfindungsgemäßen Zubereitungen, die erfindungsgemäßen Zusammensetzungen sowie die Ester gemäß der Erfindung eignen sich insbesondere in kosmetischen und/oder pharmazeutischen Zubereitungen **zur Benetzung oder Imprägnierung oder Beschichtung von Gebrauchs- und Hygienetüchern,** die zur Körperreinigung und/oder zur Körperpflege eingesetzt werden.

Als Gebrauchs- und Hygienetücher seien exemplarisch genannt: Tissues, Papiere, Wipes, Vlies-Produkte, Schwämme, Puffs, Pflaster und Bandagen, die ihren Einsatz im Bereich der Hygiene und Pflege finden. Dies können sein Feuchttücher zur Baby-Hygiene und Baby-Pflege, Reinigungstücher, Gesichtreinigungstücher, Hautpflegetücher, Pflegetücher mit Wirkstoffen gegen die Hautalterung, Wipes mit Sonnenschutzformulierungen und Insektenrepellentien sowie Wipes zur dekorativen Kosmetik oder zum After-Sun-Treatment, Toiletten-Feuchttücher, Antitranspirant-Wipes, Windeln, Taschentücher, Wet Wipes, Hygieneprodukte sowie Selbstbräunungs-Wipes.

### Beispiele

### Beispiel 1a: Herstellung 2-Propylheptylcaprylat mittels Veresterung

Es wurden 158,3 g 2-Propylheptanol (1 Mol) und 144,2 g Caprylsäure (1 Mol) zusammen mit 0,30g Fascat® 2001 (Sn-Oxalat: 0,1 % Gew.-% bezogen auf die Gesamtzusammensetzung) 5h auf 220°C am Wasserabscheider erhitzt. Nach Beendigung der Wasserabscheidung wurde das Rohprodukt im Ölpumpenvakuum über eine 250 mm Vigreuxkolonne destilliert (T = 92-94 °C). Man erhielt 282,4 g eines farblosen Öls: OHZ= < 1 SZ= <0,1.

### Beispiel 1b: Herstellung einer Zusammensetzung enthaltend 2-Propylheptylcaprylat, 4-Methyl-2-Propylhexylcaprylat und 5-Methyl-2-Propythexytcaprylat mittels Veresterung

Es wurden 158,3 g einer Mischung enthaltend 2-Propylheptanol (86%) sowie 4-Methyl-2-propylhexanol (6%) und 5-Methyl-2-propylhexanol (8%) (1 Mol) und 144,2 g Caprylsäure (1 Mol) zusammen mit 0,30g Fascat® 2001 (Sn-Oxalat: 0,1 % Gew.-% bezogen auf die Gesamtzusammensetzung) 5h auf 220°C am Wasserabscheider erhitzt. Nach Beendigung der Wasserabscheidung wurde das Rohprodukt im Ölpumpenvakuum über eine 250 mm Vigreuxkolonne destilliert (T = 92-94 °C). Man erhielt 282,4 g eines farblosen Öls: OHZ= < 1 SZ= <0,1.

### Beispiel 2a: Herstellung 2-Propylheptylcapronat mittels Veresterung

Es wurden 158,3 g 2-Propylheptanol (1 Mol) und 116,2 g Capronsäure (1 Mol) zusammen mit 0,27g Fascat® 2001 (Sn-Oxalat) (0,1 % Gew.-% bezogen auf die Gesamtzusammensetzung) 4h auf 220°C am Wasserabscheider erhitzt. Nach Beendigung der Wasserabscheidung wurde das Rohprodukt im Ölpumpenvakuum über eine 250 mm Vigreuxkolonne destilliert (T= 111-126 °C). Man erhielt 251,4 g eines farblosen Öls: OHZ= < 1 SZ= <0,1.

### Beispiel 2b: Herstellung einer Zusammensetzung enthaltend 2-Propylheptylcapronat, 4-Methyl-2-Propyl-hexylcapronat und 5-Methyl-2-Propyl-hexylcapronat mittels Veresterung

Es wurden 158,3 einer Mischung enthaltend 2-Propylheptanol (86%) sowie 4-Methyl-2-propylhexanol (6%) und 5-Methyl-2-propylhexanol (8%) (1 Mol) und 116,2 g Capronsäure (1 Mol) zusammen mit 0,27g Fascat® 2001(Sn-Oxalat) (0,1 % Gew.-% bezogen auf die Gesamtzusammensetzung) 4h auf 220°C am Wasserabscheider erhitzt. Nach Beendigung der Wasserabscheidung wurde das Rohprodukt im Ölpumpenvakuum über eine 250 mm Vigreuxkolonne destilliert (T= 111-126 °C). Man erhielt 251,4 g eines farblosen Öls: OHZ= < 1 SZ= <0,1.

### Beispiel 3a: Herstellung 2-Propylheptylcaprinat mittels Veresterung

Es wurden 1108,0g 2-Propylheptanol (7 Mol) und 1205,9 g Caprinsäure (7 Mol) zusammen mit 0,27 g Fascat® 2001 (Sn-Oxalat) (0,1 % Gew.-% bezogen auf die Gesamtzusammensetzung) 6h auf 220°C am Wasserabscheider erhitzt. Nach Beendigung der Wasserabscheidung wurde das Rohprodukt im Ölpumpenvakuum über eine 250 mm Vigreuxkolonne destilliert (T = 141-152 °C). Man erhielt 1840,1 g eines farblosen Öls: OHZ= < 0,08 SZ= <0,18.

### Beispiel 3b: Herstellung einer Zusammensetzung enthaltend 2-Propylheptylcaprinat, 4-Methyl-2-propylhexylcaprinat und 5-Methyl-2-propylhexylcaprinat mittels Veresterung

Es wurden 1108,0 g einer Mischung enthaltend 2-Propylheptanol (86%) sowie 4-Methyl-2-propylhexanol (6%) und 5-Methyl-2-propylhexanol (8%) (7 Mol) und 1205,9 g Caprinsäure (7 Mol) zusammen mit 0,27 g Fascat® 2001 (Sn-Oxalat) (0,1 % Gew.-% bezogen auf die Gesamtzusammensetzung) 6h auf 220°C am Wasserabscheider erhitzt. Nach Beendigung der Wasserabscheidung wurde das Rohprodukt im Ölpumpenvakuum über eine 250 mm Vigreuxkolonne destilliert (T =141-152 °C). Man erhielt 1840,1 g eines farblosen Öls: OHZ= < 0,08 SZ= <0,18.

### Beispiel 4a: Herstellung eines 2-Propytheptytestergemisches mittels Umesterung

1715g (10 mol) eines technischen Gemisches aus Hexansäuremethylester, Octansäuremethylester und Decansäuremethylester (Edenor ME C6-10), 1740 g (11 mol) 2-Propylheptanol und 3,6g Tetrabutyltitanat wurden unter Stickstoffatmosphäre am Wasserabscheider auf 200°C erhitzt. Nach sieben Stunden war die Reaktion beendet. Das Produkt wurde destilliert (Siedebereich: 160-175°C bei 1 mbar) und fiel als farbloses Öl an.

### Beispiel 4b: Herstellung einer Zusammensetzung enthaltend ein 2-Propylheptylestergemisch, ein 4-Methyl-2-Propylhexylestergemisch und ein 5-Methy-2-Propylhexylestergemisch mittels Umesterung

1715g (10 mol) eines technischen Gemisches aus Hexansäuremethylester, Octansäuremethylester und Decansäuremethylester (Edenor ME C6-10), 1740 g (11 mol) einer Mischung enthaltend 2-Propylheptanol (86%) sowie 4-Methyl-2-propylhexanol (6%) und 5-Methyl-2-propylhexanol (8%) und 3,6g Tetrabutyltitanat wurden unter Stickstoffatmosphäre am Wasserabscheider auf 200°C erhitzt. Nach sieben Stunden war die Reaktion beendet. Das Produkt wurde destilliert (Siedebereich: 160-175°C bei 1 mbar) und fiel als farbloses Öl an.

### Beispiel 5a: Herstellung von 2-Propylheptyloctansäureester mittels Umesterung

633,2 g (4 mol) 2-Propylheptanol, 696,2 g (4,4 mol) Octansäuremethylester (Edenor ME C8) und 22,55 g NaOCH₃ (30%ig in Methanol) wurden unter Stickstoffatmosphäre am Wasserabscheider auf 220°C erhitzt. Nach vier Stunden war die Reaktion beendet. Das Produkt Propylheptylcaprylat (= 2-Propylheptyloctanoate) wurde destilliert (Siedebereich: 106-110°C bei 0,04 mbar) und fiel als farbloses Öl an.

### Beispiel 5b: Herstellung einer Zusammensetzung enthaltend 2-Propylherptyloctansäureester, 4-Methyl-2-propylhexyl-octansäureester und 5-Methyl-2-propylhexyl-octansäureester mittels Umesterung

633,2 g (4 mol) einer Mischung enthaltend 2-Propylheptanol (86%) sowie 4-Methyl-2-propylhexanol (6%) und 5-Methyl-2-propylhexanol (8%), 696,2 g (4,4 mol) Octansäuremethylester (Edenor ME C8) und 22,55 g NaOCH₃ (30%ig in Methanol) wurden unter Stickstoffatmosphäre am Wasserabscheider auf 220°C erhitzt. Nach vier Stunden war die Reaktion beendet. Das Produkt wurde destilliert (Siedebereich: 106-110°C bei 0,04 mbar) und fiel als farbloses Öl an.

### Beispiel 6a: Enzym-katalysierte Herstellung von 2-Propylheptyl-octanoate mittels Umesterung

500 g 2-Propylheptanol, 500 g Octansäuremethylester und 50 g Novozym 435 (Fa. Novo, Dänemark; = immobilisierte Lipase - Lipase B- aus Candida antarctica) wurden unter Stickstoff 5 h auf 45°C erhitzt. Anschließend wurde die Temperatur auf 60°C erhöht und Vakuum (20mbar) angelegt. Nach 48h bei diesen Bedingungen war die Reaktion beendet. Das Produkt fiel nach dem Abfiltrieren des Enzyms als farbloses Öl an. Im GC waren nur noch weniger als 1% der Edukte zu detektieren.

### Beispiel 6b: Enzym-katalysierte Herstellung einer Zusammensetzung enthaltend 2-Propylheptyloctansäureester, 4-Methyl-2-Propylhexy-octansäureester und 5-Methyl-2-Propylhexyl-octansäureester mittels Umesterung

500 g einer Mischung enthaltend 2-Propylheptanol (86%) sowie 4-Methyl-2-propylhexanol (6%) und 5-Methyl-2-propylhexanol (8%), 500 g Octansäuremethylester und 50 g Novozym 435 (Fa. Novo, Dänemark; = immobilisierte Lipase - Lipase B- aus Candida antarctica) wurden unter Stickstoff 5 h auf 45°C erhitzt. Anschließend wurde die Temperatur auf 60°C erhöht und Vakuum (20mbar) angelegt. Nach 48h bei diesen Bedingungen war die Reaktion beendet. Das Produkt fiel nach dem Abfiltrieren des Enzyms als farbloses Öl an. Im GC waren nur noch weniger als 1 % der Edukte zu detektieren.

### Kosmetische Zubereitungen

Alle Angaben in Gew.% Aktivsubstanz bezogen auf Gesamtzubereitung.
Alle folgenden Formulierungsbeispiel wurden zum einem mit dem entsprechenden 2-Propylheptylester - wie in den Tabelle spezifiziert- hergestellt sowie zum anderen mit Zusammensetzungen enthaltend jeweils den in der Tabelle spezifizierten 2-Propylheptylester und den entsprechenden 4-Methyl-2-Propylhexylester und den entsprechenden 5-Methyl-2-Propylhexylester. Der Anteil der Summe der Methyl-2-Propylhexylester lag in den Zusammensetzungen bei 14 %, der Anteil des 2-Propylheptylesters bei 86%.

**Tabelle 1: Öl-in Wasser-Emulsionen**

| **Inhaltsstoffe Handelsname (INCI)** | **1** | **2** | **3** | **4** | **5** |
|---|---|---|---|---|---|
| Emulgade® PL 68/50 (Cetearyl Glucoside, Cetearyl Alcohol) | 4,50 | 4,50 | 4,50 | | |
| Eumulgin® VL75 (Lauryl Glucoside, Polyglyceryl-2 Dipolyhydroxystearate, Glycerin) | | | | 4,50 | 4,50 |
| 2-Propylheptylcaprinat | 16,00 | | | 16,00 | |
| 2-Propylheptylcaprylat | | 16,00 | | | 16,00 |
| 2-Propylheptylcapronat | | | 16,00 | | |
| Carbopol® 980 | | | | 0,30 | 0,30 |
| Lanette® O | | | | | |
| KOH (20%ig) | | | | 0,70 | 0,70 |
| Glycerin 99,5%ig | 3,00 | 3,00 | 3,00 | 3,00 | 3,00 |
| Formalinlsg.37%ig | 0,15 | 0,15 | 0,15 | 0,15 | 0,15 |
| Wasser dest. | ad 100 | ad 100 | ad 100 | ad 100 | ad 100 |
| pH-Wert | 7,10 | 5,90 | 5,70 | 6,70 | 6,60 |

| Viskosität (TE-Spindel mit 4 UpM , mit Helipath, 20°C) [Pa .s]: | | | | | |
|---|---|---|---|---|---|
| Tag 1 | 16800 | 16000 | 13600 | 6000 | 4000 |
| 1 Woche | 18400 | 16000 | 16000 | 6800 | 4400 |
| 4 Wochen | 20000 | 15200 | 14400 | 7600 | 5600 |
| 8 Wochen | 17600 | 14400 | 12400 | 7600 | 5600 |
| 12 Wochen | 18000 | 14000 | 13200 | 8000 | 5200 |

| Stabilitäten³⁾ | | | | | |
|---|---|---|---|---|---|
| 1 Woche RT/-5°C/40°C/45°C/50°C | 1/1/1/1/5 | 1/1/1/5/5 | 1/1/1/5/5 | 1/1/1/1/1 | 1/1/1/1/1 |
| 4 Wochen RT/-5°C/40°C/45°C/50°C | 1/1/1/1/- | 1/1/1/-/- | 1/1/1/-/- | 1/111/1/1 | 1/1/1/1/1 |
| 8 Wochen RT/-5°C/40°C/45°C/50°C | 1/1/1/5/- | 1/1/1/-/- | 1/1/1/-/- | 1/1/1/1/1 | 1/1/1/1/1 |
| 12 Wochen RT/-5°C/40°C/45°C/50°C | 1/1/1/-/- | 1/1/1/-/- | 1/1/1/-/- | 1/1/1/1/1 | 1/1/1/1/1 |

**Tabelle 2: Öl-in Wasser-Emulsionen**

| **Inhaltsstoffe: Handelsname (INCI)** | **6** | **7** | **8** | **9** |
|---|---|---|---|---|
| Eumulgin® VL75 (Lauryl Glucoside, Polyglyceryl-2 Dipolyhydroxystearate, Glycerin) | 4,50 | | | |
| Eumulgin ®B2 (Ceteareth-20) | | 2,00 | 2,00 | 2,00 |
| 2-Propylheptylcaprinat | | 16,00 | | |
| 2-Propylheptylcaprylat | | | 16,00 | |
| 2-Propylheptylcapronat | 16,00 | | | 16,00 |
| Carbopo® 980 | 0,30 | | | |
| Lanette® O | | 5,00 | 5,00 | 5,00 |
| KOH (20%ig ) | 0,70 | | | |
| Glycerin 99,5%ig | 3,00 | 3,00 | 3,00 | 3,00 |
| Formalinlsg. 37%ig | 0,15 | 0,15 | 0,15 | 0,15 |
| Wasser dest. | ad 100 | ad 100 | ad 100 | ad 100 |
| pH-Wert | 6,70 | 7,10 | 5,70 | 6,80 |

| Viskosität (TE-Spindel mit 4 UpM; mit Helipath, 20°C) [Pa.s]: | | | | |
|---|---|---|---|---|
| Tag 1 | 4000 | 11600 | 8800 | 4000 |
| 1 Woche | 4000 | 13200 | 7200 | 6400 |
| 4 Wochen | 4400 | 13600 | 7200 | 8400 |
| 8 Wochen | 4800 | 14800 | 6000 | 8400 |
| 12 Wochen | 4800 | 14400 | 6000 | 9200 |

| Stabilitäten ³⁾: | | | | |
|---|---|---|---|---|
| 1 Woche RT/-5°C/40°C/45°C/50°C | 1/1/1/1/1 | 1/1/1/5/5 | 1/1/1/5/5 | 1/1/5/5/5 |
| 4 Wochen RTl-5°C/40°Cl45°C/50°C | 1/1/1/1/1 | 1/1/1/-/- | 1/1/1/-/- | 1I1I-1-1- |
| 8 Wochen RT/-5°C/40°/C/45°C/50°C | 1/1/1/1/1 | 1/1/1/-/- | 1/1/5/-/- | 1/1/-/-/- |
| 12 Wochen RT/-5°C/40°C/45°C/50°C | 1/1/1/1/1 | 1/1/1/-/- | 1/1/5/-/- | 1/1/-/-/- |

**Tabelle 3: Wasser-in Öl-Emulsionen**

| **Inhaltsstoffe: Handelsname (INCI)** | **10** | **11** | **12** | **13** | **14** | **15** |
|---|---|---|---|---|---|---|
| Dehymuls ®LE (PEG-30-Dipolyhydroxystearate) | 5,00 | 5,00 | 5,00 | | | |
| Dehymuls® PGPH (Polyglyceryl-2-Dipo lyhydroxystearate) | | | | 4,00 | 4,00 | 4,00 |
| Lameform ®TGI (Polyglyceryl-3-Diisostearate) | | | | 2,00 | 2,00 | 2,00 |
| 2-Propylheptyldecanoat | 20,00 | | | 20,00 | | |
| 2-Propylheptyloctanoat | | 20,00 | | | 20,00 | |
| 2-Propylheptylhexanoat | | | 20,00 | | | 20,00 |
| MgSO4*7H2O | 1,00 | 1,00 | 1,00 | 1,00 | 1,00 | 1,00 |
| Glycerin 99,5%ig | 5,00 | 5,00 | 5,00 | 5,00 | 5,00 | 5,00 |
| Formalinlsg. 37%ig | 0,15 | 0,15 | 0,15 | 0,15 | 0,15 | 0,15 |
| Wasser dest. | Ad 100 | ad 100 | ad 100 | ad 100 | ad 100 | ad 100 |

| Viskosität (Brookfield RVF, TE-Spindel mit 4 Upm, 20°C) [Pa.s]: | | | | | | |
|---|---|---|---|---|---|---|
| Tag 1 | 6000 | 5200 | 3600 | 16000 | 14400 | 9600 |
| 1 Woche | 6800 | 6000 | 4000 | 18000 | 16400 | 11200 |
| 4 Wochen | 6800 | 6000 | 4000 | 20000 | 19600 | 14400 |
| 8 Wochen | 6800 | 5600 | 4000 | 21600 | 21600 | 15200 |
| 12 Wochen | 6800 | 5600 | 4000 | 21200 | 22000 | 14800 |

| Stabilitäten³): | | | | | | |
|---|---|---|---|---|---|---|
| 1 Woche RT/-5°C/40°C/ 45°C/50°C | 1/1/1/1/1 | 1/1/1/1/1 | 1/1/1/1/1 | 1/1/1/1/1 | 1/1/1/1/1 | 1/1/1/1/1 |
| 4 Wochen RT/-5°C/40°C/ 45°C/50°C | 1/1/1/1/1 | 1/1/1/1/1 | 1/1/1/1/1 | 1/1/1/1/1 | 1/1/1/1/1 | 1/1/1/1/1 |
| 8 Wochen RT/-5°C/40°C/ 45°C/50°C | 1/1/1/1/1 | 1/1/1/1/1 | 1/1/1/1/1 | 1/1/1/1/1 | 1/1/1/1/1 | 1/1/1/1/1 |
| 12 Wochen RT/-5°C/40°C/ 45°C/50°C | 1/1/1/1/1 | 1/1/1/1/1 | 1/1/1/1/1 | 1/1/1/1/1 | 1/1/1/1/1 | 1/1/1/1/1 |

**Tabelle 4: Wasser-in Öl-Emulsionen**

| **Inhaltsstoffe Handelsname (INCI)** | **16** | **17** | **18** |
|---|---|---|---|
| Dehymuls ®LE (PEG-30-Dipolyhydroxystearate) | 4,00 | 4,00 | 4,00 |
| Lameform ®TGI (Polyglyceryl-3-Diisostearate) | 2,00 | 2,00 | 2,00 |
| 2-Propylheptyldecanoat | 20,00 | | |
| 2-Propylheptyloctanoat | | 20,00 | |
| 2-Propylheptylhexanoat | | | 20,00 |
| MgSO4*7H2O | 1,00 | 1,00 | 1,00 |
| Glycerin 99,5%ig | 5,00 | 5,00 | 5,00 |
| Formalinlsg. 37%ig | 0,15 | 0,15 | 0,15 |
| Wasser dest. | ad 100 | ad 100 | ad 100 |

| Viskosität (Brookfield RVF, TE-Spindel mit 4 Upm, 20°C) [Pa s]: | | | |
|---|---|---|---|
| Tag 1 | 18000 | 16000 | 14000 |
| 1 Woche | 22400 | 23600 | 17600 |
| 4 Wochen | 23200 | 20000 | 17600 |
| 8 Wochen | 22800 | 19600 | 16800 |
| 12 Wochen | 22800 | 20000 | 16800 |

| Stabilitäten | | | |
|---|---|---|---|
| **Inhaltsstoffe Handelsname (INCI)** | **16** | **17** | **18** |
| 1 Woche RT/-5°C/40°C/45°C/50°C | 1/1/1/1/1 | 1/1/1/1/1 | 1/1/1/1/1 |
| 4 Wochen RT/-5°C/40°C/45°C/50°C | 1/1/1/1/1 | 1/1/1/1/1 | 1/1/1/1/1 |
| 8 Wochen RT/-5°C/40°C/45°C/50°C | 1/1/1/1/1 | 1/1/1/1/1 | 1/1/1/1/1 |
| 12 Wochen RT/-5°C/40°C/45°C/50°C | 1/1/1/1/1 | 1/1/1/1/1 | 1/1/1/1/1 |

### Weitere Formulierungs-Beispiele

| Beispiel **19:** Hair Conditioner | | Beispiel **20:** Nanoemulsion | |
|---|---|---|---|
| Dehyquart® A CA (Cetrimonium Chloride) | 4.5% | Monomuls® 90 O 18 (Glyceryl Oleate) | 6,11 % |
| Lanette®O (Cetearyl Alcohol) | 4% | 2-Propylheptylcarpylat | 17,88 % |
| Cutina ®CP (Cetyl Palmitate) | 1% | Eutanol®G (Octyldodecanol) | 5,97 % |
| 2-Propylheptylcaprylate | 1.5% | Plantapon®LGC Sorb (Sodium Lauryl Glucose | 9,5 % |
| Eumulgin ®B2 (Ceteareth-20) | 0.3% | Carboxylate (and) Lauryl Glucoside) | |
| Konservierungsmittel | q.s. | | |
| Aqua demin. | ad 100 | Plantapon® ACG 35 (Disodium Cocoyl Glutamate) | 0,78 % |
| | | Phenoxyethanol | 0,5 % |
| | | Phenonip | 0,5 % |
| | | Aqua demin. | ad 100 |

**Tabelle 5**

| **Handelsname (INCI)** | **21** | **22** | **23** | **V1** | **24** | **25** | **26** | **27** |
|---|---|---|---|---|---|---|---|---|
| Emulgade® SE-PF (Glyceryl Stearate, Ceteareth-20, Ceteareth-12, Cetearyl Alcohol, Cetyl Palmitate) | 4,80 | | | | | | | |
| Eumulgin® B2 (Ceteareth-20) | 3,70 | | 3,00 | 3,00 | | | | |
| Emulgade® PL-68/50 (Cetearyl Glucoside, Cetearyl Alcohol) | | | | | 5,00 | | | |
| Eumulgin® SG (Sodium Stearoyl Glutamate) | | | | | 0,50 | 0,20 | | |
| Eumulgin® VL 75 (Lauryl Glucoside, Polyglyceryl-2 Dipolyhydroxystearate, Glycerin) | | 6,00 | | | | | | 0,50 |
| Cutina® MD (Glyceryl Stearate) | | | | | 2,00 | | | |
| Cutina® PES (Pentaerythrityl Distearate) | | | | | | 1,00 | | |
| Cetiol® SenSoft (Propylheptyl Caprylate¹)) | 5,00 | 7,00 | 2,00 | | 5,00 | 5,00 | 5,00 | 6,00 |
| Cetiol® 868 (Ethylhexyl Stearate) | | | 7 , 00 | 7,00 | 4,00 | | | |
| Cetiol® AB (C12-15 Alkyl Benzoate) | | 7,00 | | | | | | |
| Cetiol® LC (Coco-Caprylate / Caprate) | | | | | | 5,00 | 5,00 | |
| Myritol® 331 (Cocoglycerides) | 3,00 | | | | | | | 10,00 |
| Myritol® 312 (Caprylic / Capric Triglyceride) | | | | | 5, 00 | | | |
| Myritol® 318 (Caprylic / Capric Triglyceride) | | | 7,00 | 7,00 | | | | |
| Dimethicone (Wacker AK 350) | | | | | 0,50 | | | |
| Ethylhexyl Methoxycinnamate (Uvinul MC 80) | 5,00 | 7,50 | | | | | | 7,50 |
| 4-Methylbenzylidene Camphor (Neo Helipan MBC) | 2,00 | | | | | | | |
| Butyl Methoxydibenzoylmethane (Parsol 1789) | 1,50 | 3,50 | | | | | | 2,00 |
| Copherol® F 1300 C (Tocopherol) | | | | | 1,00 | | | 1,00 |
| Cosmedia® DC (Hydrogenated Dimer Dilinoleyl/Dimethylcarbonate Copolymer) | | | | | | | | 2,00 |
| Cosmedia® SP (Sodium Polyacrylate) | | 0,50 | 0,20 | 0,20 | | 1,00 | 1,00 | 0,30 |
| Glycerin | 5,00 | | | | 2,00 | 5,00 | | 5,00 |
| 1,3-Butylene Glycol | | 3,00 | | | 2,00 | | | |
| Phenylbenzimidazole Sulfonic Acid (Neo Heliopan Hydro, 15% wässrige Lösung) | 13,30 | | | | | | | |
| Methylene Bis-Benzotriazolyl Tetramethylbutylphenol (Tinosorb M) | | 5,00 | | | | | | |

| **Handelsname (INCI)** | **21** | **22** | **23** | **V1** | **24** | **25** | **26** | **27** |
|---|---|---|---|---|---|---|---|---|
| Tapioca Stärke | | | | 2,00 | | | | |
| Wasser, Konservierungsmittel q.s. | Ad100 | | | | | | | |
| NaOH (10%) | pH 7,0 | pH 6,6 | pH 6,3 | pH 6,3 | pH 7,0 | pH 6,1 | pH 6,5 | pH 6,0 |

| Viskositätsdaten²) bei 20 °C [Pa·s) | | | | | | | | |
|---|---|---|---|---|---|---|---|---|
| nach Vorbereitung | <400 | <400 | 2000 | 1200 | 87500 | 187500 | 112500 | 12800 |
| nach 1 Woche | <400 | <400 | 2000 | 1200 | 62500 | 162500 | 112500 | 12400 |
| nach 2 Wochen | <400 | <400 | 2400 | 1200 | 62500 | 162500 | 112500 | 11600 |
| nach 4 Wochen | <400 | | 2400 | 1200 | 62500 | 150000 | | |
| nach 8 Wochen | <400 | | 2800 | 1600 | 62500 | 162500 | | |

| Phasenstabilität³⁾ bei -5°C/20 °C /40°C | | | | | | | | |
|---|---|---|---|---|---|---|---|---|
| nach 1 Woche | 1/1/1 | 1/1/1 | 1/1/1 | 1/1/1 | 1/1/1 | 1/1/1 | 1/1/1 | 1/1/1 |
| nach 2 Wochen | 1/1/1 | 1/1/1 | 1/1/1 | 1/1/1 | 1/1/1 | 1/1/1 | 1/1/1 | 1/1/1 |
| nach 4 Wochen | 1/1/1 | | 1/1/1 | 1/1/1 | 1/1/1 | 1/1/1 | | |
| nach 8 Wochen | 1/1/1 | | 1/1/5 | 1/1/1 | 1/1/1 | 1/1/1 | | |
| | | | | | | | | |
| Makroskopische⁴)/ Mikroskopische⁵) Erscheinung bei 20 °C | -/1 | 2/2 | 2/2 | 2/2 | 2/3 | 2/2 | 2/3 | 1/3 |

| Sensorische Bewertung⁶) | | | | | | | | |
|---|---|---|---|---|---|---|---|---|
| Weichheit | 1 | 1 | 1 | 3 | 1 | 1 | 1 | 1 |
| Glätte | 1 | 1 | 2 | 1 | 2 | 1 | 1 | 2 |

**Tabelle 6**

| **Handelsname (INCI)** | **28** | **29** | **30** | **31** |
|---|---|---|---|---|
| Dehymuls® PGPH (Polyglyceryl-2 Dipolyhydroxystearate) | 2,00 | 2,00 | | |
| Dehymuls® LE (PEG-30 Dipolyhydroxystearate) | | 2,00 | | |
| Cyclopentasiloxane, Caprylyl Dimethicone, EthoxyGlucoside (WackerBelsil SPG 128 VP) | 12,00 | | | |
| Bienenwachs 8100 (Kahl) | 1,00 | | | |
| Zinkstearat (Zinkum N 29) | 1,00 | | | |
| Texapon® NSO (Sodium Laureth Sulfate) | | | | 34,00 |
| Dehyton® PK 45 (Cocamidopropyl Betaine) | | | | 8,00 |
| Emulgade® NLB (Steareth-2, Ceteareth-12, Stearyl Alcohol, Ceteareth-20, Distearyl Ether) | | | | 3,00 |
| Polyquaternium-10 (Polymer JR 400) | | | | 0,20 |
| Acrylates Copolymer (Carbopol Aqua SF-1) | | | | 8,00 |
| Cetiol® SenSoft (Propylheptyl Caprylate¹⁾) | 8,00 | 6,00 | 10,00 | 3,00 |
| Cetiol® 868 (Ethylhexyl Stearate) | 7,00 | | | |
| Cetiol® A (Hexyl Laurate) | | 6,00 | | |
| Cetiol® SN (Cetearyl Isononanoate) | | 7,00 | | |
| Eutanol® G 16 (Hexyldecanol) | | 3,00 | | |
| Myritol® 331 (Cocoglycerides) | | | 31,00 | |
| Helianthus Annuus (Sonnenblumenöl) | | | 57,00 | |
| Copherol® 1250 C (Tocopheryl Acetate) | | | 1,00 | |
| Copherol® F 1300 C (Tocopherol) | 1,00 | | | |
| Glycerin | | 5,00 | | |
| 1,3-Butylene Glycol | 3,00 | | | |
| Sodium Chloride | 0,40 | | | |
| Magnesium Sulfate Heptahydrate | | 1,00 | | |
| Alcohol (Ethanol) | | 4,00 | | |
| Hydagen® B (Bisabolol) | | | 0,50 | |
| Wasser, Konservierungsmittel q.s. | ad 100 | ad 100 | | ad 100 |

| Viskositätsdaten²⁾ bei 20 °C [Pa·s) | | | | |
|---|---|---|---|---|
| nach Vorbereitung | 11200 | 2400 | <400 | 3000 |
| nach 1 Woche | 20000 | 2400 | <400 | 3400 |
| nach 2 Wochen | 20000 | 2400 | <400 | 3400 |
| nach 4 Wochen | 20000 | | <400 | 3600 |
| nach 8 Wochen | 19600 | | | |

| Phasenstabilität³) bei -5°C/20 °C /40°C | | | | |
|---|---|---|---|---|
| nach 1 Woche | 1/1/1 | 1/1/1 | 1/1/1 | 1/1/1 |
| nach 2 Wochen | 1/1/1 | 1/1/1 | 1/1/1 | 1/1/1 |
| nach 4 Wochen | 1/1/1 | | 1/1/1 | 1/1/1 |
| nach 8 Wochen | 1/1/1 | | | |
| Makroskopische⁴⁾/ Mikroskopische⁵⁾ Erscheinung bei 20 °C | 1/2 | 1/1 | n.d. | n.d. |

| Sensorische Bewertung⁶⁾ | | | | |
|---|---|---|---|---|
| Weichheit | 1 | 1 | 1 | 1 |
| Glätte | 1 | 1 | 1 | 1 |

**Tabelle 7**

| **Handelsname (INCI)** | **32** | **33** | **34** |
|---|---|---|---|
| Emulgade® NLB (Steareth-2, Ceteareth-12, Stearyl Alcohol, Ceteareth-20, Distearyl Ether) | 5,00 | 5,00 | |
| Lanette® 18 (Stearyl Alcohol) | | | 14,70 |
| Cutina® HR (Hydrogenated Castor Oil) | | | 3,70 |
| Cetiol® SenSoft (Propylheptyl Caprylate¹⁾) | 6,00 | 4,50 | 23,70 |
| Cyclomethicone (Dow Corning 245) | | 1,50 | 35,00 |
| Aluminium Chlorohydrate (Chlorhydrol 50%ig) | 40,00 | 20,00 | |
| Aluminium Zirconium Tetrachlorohydrex GLY (Rezal 36 GP) | | | 22,90 |
| Wasser | ad 100 | ad 100 | |
| | | | |

| Viskositätsdaten²⁾ bei 20 °C [Pa·s] | | | |
|---|---|---|---|
| nach Vorbereitung | <400 | 440 | |
| nach 1 Woche | 2000 | 5600 | |
| nach 2 Wochen | 2000 | 5200 | |
| nach 4 Wochen | 2800 | 4800 | |
| nach 8 Wochen | 2800 | 4800 | |
| nach 12 Wochen | 3200 | | |

| Härte | | | |
|---|---|---|---|
| nach 1 Tag | | | 3,3 |
| nach 12 Wochen | | | 3,5 |

| Phasenstabilität³⁾ bei -5°C/20 °C 140°C | | | |
|---|---|---|---|
| nach 1 Woche | 1/1/1 | 1/1/1 | 1/1/1 |
| nach 2 Wochen | 1/1/1 | 1/1/1 | |
| nach 4 Wochen | 1/1/1 | 1/1/1 | 1/1/1 |
| nach 8 Wochen | 1/1/2 | 1/1/1 | 1/1/1 |
| Nach 12 Wochen | 1/1/2 | | 1/1/1 |

| **Handelsname (INCI)** | **32** | **33** | **34** |
|---|---|---|---|
| Makroskopische⁴⁾/ Mikroskopische⁵⁾ Erscheinung bei 20 °C | 2/1 | 2/2 | n.a. |
| Fussnoten zu Tabellen: | | | |
| RT = Raumtemperatur 20°C; Upm= Umdrehungen pro Minute | | | |
| 1) Proposed INCI. | | | |
| 2) Viskositätsmessungen: Brookfield RVF, Spindel 5, 10 Umdrehungen pro Minute, 23°C (Formulierungen 21, 22, 23, V1 und 27, 28 bis 34) bzw. Brookfield RVF, Spindel TE mit Helipath, 4 Umdrehungen pro Minute, 23 °C (Formulierungen 24, 25 und 26). | | | |
| 3) Bewertungskriterien für die visuelle Phasenstabilität: 1 = stabil; 2 = geringe Separationen; 3 = Separationen; 4 = deutliche Separationen; 5 = Trennungen. | | | |
| 4) Bewertungskriterien zum visuellen makroskopischen Erscheinungsbild: 1 = glatt und glänzend; 2 = glatt und matt; 3 = matt / grobe Struktur; 4 = sichtbare Rekristallisate. Die Bewertung der Formulierungen erfolgte nach temperieren auf RT. | | | |
| 5) Bewertungskriterien zum mikroskopischen Erscheinungsbild: 1 = durchschnittliche Teilchengröße ≤ 1µm; 2 = durchschnittliche Teilchengröße 1 - 4µm; 3 = durchschnittliche Teilchengröße 4 - 13µm; 4 = durchschnittliche Teilchengröße 13 - 20µm; 5= durchschnittliche Teilchengröße 20 - 50µm. Die Partikelgröße der Testemulsion wurde visuell mit der Partikelgröße von Standardemulsionen verglichen. Zur Bestimmung der Partikelgröße der Standardemulsionen wird mittels Laserbeugung ein Beugungsmuster ermittelt. Aus den Lichtintensitäten dieser Beugungsmuster wird dann mittels der Fraunhofer-Theorie die Teilchengrößenverteilung errechnet (Sympatec Helos). | | | |
| 6) Sensorische Bewertungskriterien: | | | |
| Die Sensorische Bewertung wurde wie folgt durchgeführt: | | | |
| Testgruppe: 10 erfahrene und geschulte Freiwillige; 1 = sehr hohe Akzeptanz; 2 = durchschnittliche Akzeptanz; 3 = nicht akzeptabel | | | |
| 10µl der o.g. Zusammensetzungen, die vorher auf 20 °C gebracht wurden, wurden mittels einer Mikropipette auf die unbehaarte Seite der Unterarme der Probanden appliziert und mit den Fingern der Hände der kontralateralen Seite eingerieben. Die Beurteilung der Sensorik erfolgte während und nach der Absorption. Der Sensorik-Test wurde an 10 Probanden durchgeführt, wie in dem Buch "Cosmetic Lipids and the Skin Barrier" (Marcel Dekker Verlag New York, 2002, Ed. Thomas Förster, S. 319-352) beschrieben. | | | |

## Patentansprüche

1. Verwendung von Estern des 2-Propylheptanols mit linearen oder verzweigten, gesättigten oder ungesättigten C4-C36-Carbonsäuren - oder C4-C36-Dicarbonsäuren in kosmetischen und/oder pharmazeutischen Zubereitungen.

2. Ester von 2-Propylheptanol mit Carbonsäuren ausgewählt aus linearen, verzweigten, gesättigten oder ungesättigten C4 bis C36 Carbonsäuren, mit Ausnahme von 2-Propylheptyl methacrylat.

3. Ester nach Anspruch 2, ausgewählt aus der Gruppe bestehend aus 2-Propylheptyl-n-butansäureester, 2-Propylheptyl-i-butansäureester, 2-Propylheptyl-n-pentansäureester, 2-Propylheptyl-i-pentansäureester, 2-Propylheptyl-n-hexansäureester, 2-Propylheptyl-i-hexansäureester, 2- Propylheptyl-n-heptansäureester, 2- Propylheptyl-i-heptansäureester, 2-Propylheptyl-n-octansäureester, 2-Propylheptyl-i-octansäureester, 2- Propylheptyl-n-nonansäureester, 2- Propylheptyl-i-nonansäureester, 2-Propylheptyl-n-decansäureester, 2-Propylheptyl-i-decansäureester, 2-Propylheptyl-n-undecansäureester, 2-Propylheptyl-i-undecansäureester, 2-Propylheptyl-n-undecensäureester, 2-Propylheptyl-n-dodecansäureester, 2-Propylheptyl-i-dodecansäureester.

4. Verfahren zur Herstellung der Ester gemäß einem der vorgenannten Ansprüche, wobei man eine Mischung enthaltend 2-Propylheptanol und die entsprechende Säure umsetzt.

5. Verfahren zur Herstellung der Ester gemäß Anspruch 3, wobei man eine Mischung enthaltend 2-Propylheptanol und den Methylester der entsprechenden Säure unter Zusatz eines Umesterungskatalysators umsetzt.

6. Kosmetische und/oder pharmazeutische Zusammensetzungen enthaltend
(a) wenigstens einen Ester des 2-Propylheptanols mit linearen oder verzweigten, gesättigten oder ungesättigten C4-C36-Carbonsäuren - oder C4-C36-Dicarbonsäuren, vorzugsweise wenigstens einen Ester des 2-Propylheptanols mit linearen oder verzweigten, gesättigten oder ungesättigten C4-C18-Carbonsäuren -oder C4-C18-Dicarbonsäuren
(b) wenigstens einen Emulgator (b-1) und/oder Tensid (b-2) und/oder Wachskomponente (b-3) und/oder Polymer (b-4) und/oder einen weiteren Ölkörper (b-5)

7. Zusammensetzung gemäß Anspruch 6, enthaltend 0,1 - 80 Gew.-%, insbesondere 0,1 bis 70, bevorzugt 0,1 bis 60, insbesondere 0,1 bis 50 Gew.-%, bevorzugt 0,1 - 40 Gew.-% wenigstens eines Ester des 2-Propylheptanols mit linearen oder verzweigten, gesättigten oder ungesättigten C4-C36-Carbonsäuren - oder C4-C36-Dicarbonsäuren, vorzugsweise wenigstens eines Esters des 2-Propylheptanols mit linearen oder verzweigten, gesättigten oder ungesättigten C4-C18-Carbonsäuren - oder C4-C18-Dicarbonsäuren.

8. Zusammensetzung gemäß Anspruch 6 und/oder 7 enthaltend
a) 0,1 - 80 Gew.-%, insbesondere 0,1 bis 70, bevorzugt 0,1 bis 60, insbesondere 0,1 bis 50 Gew.-%, bevorzugt 0,1 - 40 Gew.-% wenigstens eines Ester des 2-Propylheptanols mit linearen oder verzweigten, gesättigten oder ungesättigten C4-C36-Carbonsäuren - oder C4-C36-Dicarbonsäuren, vorzugsweise wenigstens eines Esters des 2-Propylheptanols mit linearen oder verzweigten, gesättigten oder ungesättigten C4-C18-Carbonsäuren - oder C4-C18-Dicarbonsäuren
b) 0,1 - 20 Gew.-% Emulgator (b-1) und/oder Tensid (b-2) und/oder Wachskomponente
(b-3) und/oder Polymer (b-4)
(b-5) 0,1 - 40 Gew.-% weiterer Ölkörper und
c) 0- 98 Gew.-% Wasser.

9. Zusammensetzungen gemäß einem der Ansprüche 6 bis 8, enthaltend 2-Propyl-heptyl-n-butansäureester, 2-Propyl-heptyl-i-butansäureester, 2- Propylheptyl-n-pentansäureester, 2-Propylheptyl-i-pentansäureester, 2-Propylheptyl-n-hexansäureester, 2-Propylheptyl-i-hexansäureester, 2-Propylheptyl-n-heptansäureester, 2-Propylheptyl-i-heptansäureester, 2-Propyl-heptyl-n-octansäureester, 2-Propylheptyl-i-octansäureester, 2-Propylheptyl-n-nonansäureester, 2-Propylheptyl-i-nonansäureester, 2-Propylheptyl-n-decansäureester, 2-Propylheptyl-i-decansäureester, 2-Propylheptyl-n-undecansäureester, 2-Propylheptyl-i-undecansäureester, 2-Propylheptyl-n-undecensäureester, 2-Propylheptyl-n-dodecansäureester, 2-Propylheptyl-i-dodecansäureester oder ein beliebiges Gemisch dieser Substanzen.

## Claims

1. Use of esters of 2-propylheptanol with linear or branched, saturated or unsaturated C4-C36-carboxylic acids - or C4-C36-dicarboxylic acids in cosmetic and/or pharmaceutical preparations.

2. Esters of 2-propylheptanol with carboxylic acids selected from linear, branched, saturated or unsaturated C4 to C36 carboxylic acids, with the exception of 2-propylheptyl methacrylate.

3. Esters according to Claim 2 selected from the group consisting of 2-propylheptyl n-butanoate, 2-propylheptyl isobutanoate, 2-propylheptyl n-pentanoate, 2-propylheptyl isopentanoate, 2-propylheptyl n-hexanoate, 2-propylheptyl isohexanoate, 2-propyl-heptyl n-heptanoate, 2-propylheptyl isoheptanoate, 2-propylheptyl n-octanoate, 2-propylheptyl isooctanoate, 2-propylheptyl n-nonanoate, 2-propyl-heptyl isononanoate, 2-propylheptyl n-decanoate, 2-propylheptyl isodecanoate, 2-propylheptyl n-undecanoate, 2-propylheptyl isoundecanoate, 2-propyl-heptyl n-undecenoate, 2-propylheptyl n-dodecanoate, 2-propylheptyl isododecanoate.

4. Process for the preparation of the esters according to one of the aforementioned claims, where a mixture comprising 2-propylheptanol and the corresponding acid is reacted.

5. Process for the preparation of the esters according to Claim 3, where a mixture comprising 2-propylheptanol and the methyl ester of the corresponding acid is reacted with the addition of a transesterification catalyst.

6. Cosmetic and/or pharmaceutical compositions comprising
(a) at least one ester of 2-propylheptanol with linear or branched, saturated or unsaturated C4-C36-carboxylic acids - or C4-C36-dicarboxylic acids, preferably at least one ester of 2-propylheptanol with linear or branched, saturated or unsaturated C4-C18-carboxylic acids - or C4-C18-dicarboxylic acids
(b) at least one emulsifier (b-1) and/or surfactant (b-2) and/or wax component (b-3) and/or polymer (b-4) and/or a further oil body (b-5).

7. Composition according to Claim 6, comprising 0.1-80% by weight, in particular 0.1 to 70, preferably 0.1 to 60, in particular 0.1 to 60% by weight, preferably 0.1-40% by weight, of at least one ester of 2-propylheptanol with linear or branched, saturated or unsaturated C4-C36-carboxylic acids - or C4-C36-dicarboxylic acids, preferably at least one ester of 2-propylheptanol with linear or branched, saturated or unsaturated C4-C18-carboxylic acids - or C4-C18-dicarboxylic acids.

8. Composition according to Claim 6 and/or 7, comprising
a) 0.1-80% by weight, in particular 0.1 to 70, preferably 0.1 to 60, in particular 0.1 to 50% by weight, preferably 0.1-40% by weight, of at least one ester of 2-propylheptanol with linear or branched, saturated or unsaturated C4-C36-carboxylic acids - or C4-C36-dicarboxylic acids, preferably at least one ester of 2-propylheptanol with linear or branched, saturated or unsaturated C4-C18-carboxylic acids - or C4-C18-dicarboxylic acids
b) 0.1-20% by weight of emulsifier (b-1) and/or surfactant (b-2) and/or wax component (b-3) and/or polymer (b-4)
b-5) 0.1-40% by weight of further oil bodies and
c) 0-98% by weight of water.

9. Compositions according to one of Claims 6 to 8, comprising 2-propylheptyl n-butanoate, 2-propylheptyl isobutanoate, 2-propylheptyl n-pentanoate, 2-propylheptyl isopentanoate, 2-propylheptyl n-hexanoate, 2-propylheptyl isohexanoate, 2-propyl-heptyl n-heptanoate, 2-propylheptyl isoheptanoate, 2-propylheptyl n-octanoate, 2-propylheptyl isooctanoate, 2-propylheptyl n-nonanoate, 2-propylheptyl isononanoate, 2-propylheptyl n-decanoate, 2-propyl-heptyl isodecanoate, 2-propylheptyl n-undecanoate, 2-propylheptyl isoundecanoate, 2-propylheptyl n-undecenoate, 2-propylheptyl n-dodecanoate, 2-propyl-heptyl isododecanoate or any desired mixture of these substances.

## Revendications

1. Utilisation d'esters du 2-propylheptanol avec des acides carboxyliques en C₄-C₃₆ - ou acides dicarboxyliques en C₄-C₃₆, linéaires ou ramifiés, saturés ou insaturés, dans des préparations cosmétiques et/ou pharmaceutiques.

2. Esters de 2-propylheptanol avec des acides carboxyliques choisis parmi des acides carboxyliques en C₄-C₃₆ linéaires, ramifiés, saturés ou insaturés, à l'exception du méthacrylate de 2-propylheptyle.

3. Esters selon la revendication 2, choisis dans le groupe constitué par le n-butanoate de 2-propylheptyle, l'isobutanoate de 2-propylheptyle, le n-pentanoate de 2-propylheptyle, l'isopentanoate de 2-propylheptyle, le n-hexanoate de 2-propylheptyle, l'isohexanoate de 2-propylheptyle, le n-heptanoate de 2-propylheptyle, l'isoheptanoate de 2-propylheptyle, le n-octanoate de 2-propylheptyle, l'iso-octanoate de 2-propylheptyle, le n-nonanoate de 2-propylheptyle, l'isononanoate de 2-propylheptyle, le n-décanoate de 2-propylheptyle, l'isodécanoate de 2-propylheptyle, le n-undécanoate de 2-propylheptyle, l'iso-undécanoate de 2-propylheptyle, le n-undécénoate de 2-propylheptyle, le n-dodécanoate de 2-propylheptyle, l'isododécanoate de 2-propylheptyle.

4. Procédé pour la préparation des esters selon l'une quelconque des revendications précédentes, dans lequel on fait réagir un mélange contenant du 2-propylhexanol et l'acide correspondant.

5. Procédé pour la préparation des esters selon la revendication 3, dans lequel on fait réagir un mélange contenant du 2-propylheptanol et l'ester méthylique de l'acide correspondant, avec addition d'un catalyseur de transestérification.

6. Compositions cosmétiques et/ou pharmaceutiques contenant
(a) au moins un ester du 2-propylheptanol avec des acides carboxyliques en C₄-C₃₆ - ou acides dicarboxyliques en C₄-C₃₆, linéaires ou ramifiés, saturés ou insaturés, de préférence au moins un ester du 2-propylheptanol avec des acides carboxyliques en C₄-C₁₈ - ou acides dicarboxyliques en C₄-C₁₈, linéaires ou ramifiés, saturés ou insaturés,
(b) au moins un émulsifiant (b-1) et/ou agent tensioactif (b-2) et/ou composant cireux (b-3) et/ou polymère (b-4) et/ou un autre corps huileux (b-5) .

7. Composition selon la revendication 6, contenant 0,1 - 80 % en poids, en particulier 0,1 à 70, de préférence 0,1 à 60, en particulier 0,1 à 60 % en poids, de préférence 0,1 - 40 % en poids d'au moins un ester du 2-propylheptanol avec des acides carboxyliques en C₄-C₃₆ - ou acides dicarboxyliques en C₄-C₃₆, linéaires ou ramifiés, saturés ou insaturés, de préférence d'au moins un ester du 2-propylheptanol avec des acides carboxyliques en C₄-C₁₈ - ou acides dicarboxyliques en C₄-C₁₈, linéaires ou ramifiés, saturés ou insaturés.

8. Composition selon la revendication 6 et/ou la revendication 7, contenant
a) 0,1 - 80 % en poids, en particulier 0,1 à 70, de préférence 0,1 à 60, en particulier 0,1 à 50 % en poids, de préférence 0,1 - 40 % en poids d'au moins un ester du 2-propylheptanol avec des acides carboxyliques en C₄-C₃₆ - ou acides dicarboxyliques en C₄-C₃₆, linéaires ou ramifiés, saturés ou insaturés, de préférence d'au moins un ester du 2-propylheptanol avec des acides carboxyliques en C₄-C₁₈ - ou acides dicarboxyliques en C₄-C₁₈, linéaires ou ramifiés, saturés ou insaturés,
b) 0,1 - 20 % en poids d'émulsifiant (b-1) et/ou d'agent tensioactif (b-2) et/ou de composant cireux (b-3) et/ou de polymère (b-4)
(b-5)0,1 - 40 % en poids d'autres corps huileux et
c) 0 - 98 % en poids d'eau.

9. Compositions selon l'une quelconque des revendications 6 à 8, contenant du n-butanoate de 2-propylheptyle, de l'isobutanoate de 2-propylheptyle, du n-pentanoate de 2-propylheptyle, de l'isopentanoate de 2-propylheptyle, du n-hexanoate de 2-propylheptyle, de l'isohexanoate de 2-propylheptyle, du n-heptanoate de 2-propylheptyle, de l'isoheptanoate de 2-propylheptyle, du n-octanoate de 2-propylheptyle, de l'iso-octanoate de 2-propylheptyle, du n-nonanoate de 2-propylheptyle, de l'isononanoate de 2-propylheptyle, du n-décanoate de 2-propylheptyle, de l'isodécanoate de 2-propylheptyle, du n-undécanoate de 2-propylheptyle, de l'iso-undécanoate de 2-propylheptyle, du n-undécénoate de 2-propylheptyle, du n-dodécanoate de 2-propylheptyle, de l'isododécanoate de 2-propylheptyle ou un mélange quelconque de ces substances.
